# EUROPEAN PATENT SPECIFICATION

(11) **EP 2 705 031 B1**
(45) Date of publication and mention of the grant of the patent: **11.04.2018**
(21) Application number: 12721076.3
(22) Date of filing: 30.04.2012
(51) Int. Cl.: C07D 321/00, C07D 407/12

(54) **FLASH AND GLOW 1,2-DIOXETANES**
FLASH- UND GLOW-1,2-DIOXETANE
1,2-DIOXÉTANES INCANDESCENTS ET LUMINESCENTS

(30) Priority: 03.05.2011 US 201161482134 P
(43) Date of publication of application: 12.03.2014
(73) Proprietor: Life Technologies Corporation, Carlsbad, CA 92008 (US)
(72) Inventor: MIHALI, Albana, Carlsbad California 92008 (US); EDWARDS, Brooks, Cambridge Massachusetts 02138 (US); WANG, Zhixian, Winchester Massachusetts 01890 (US)
(74) Representative: Hoffmann Eitle
(86) International application number: PCT/US2012/035732
(87) International publication number: WO 2012/151142

(56) References cited:
- WO-A2-2006/073424
- WO-A2-2010/101839
- US-A- 5 780 249
- US-A1- 2006 079 699
- AKHAVAN-TAFTI H ET AL: "A novel substitution process for the preparation of alkoxy-, aryloxy-, and acyloxy-substituted 1,2-dioxetanes", JOURNAL OF THE AMERICAN CHEMICAL SOCIETY, AMERICAN CHEMICAL SOCIETY, WASHINGTON, DC; US, vol. 119, 1 January 1997 (1997-01-01), pages 245-246, XP002370651, ISSN: 0002-7863, DOI: 10.1021/JA962245A
- MATSUMOTO M ET AL: "Synthesis of 3-Alkoxymethyl-4-aryl-3-tert-butyl-4-metho xy-1,2-dioxetanes as a Chemiluminescent Substrate with Short Half-life Emission", TETRAHEDRON LETTERS, ELSEVIER, AMSTERDAM, NL, vol. 37, no. 33, 12 August 1996 (1996-08-12), pages 5939-5942, XP004030579, ISSN: 0040-4039, DOI: 10.1016/0040-4039(96)01282-8
- ROCHE APPLIED SCIENCE: "CSPD, ready-to-use", 20030501, 1 May 2003 (2003-05-01), pages 1-4, XP007922996,

## Description

### FIELD OF THE INVENTION

This invention relates to detection reagents for diagnostic platforms.

### BACKGROUND OF THE INVENTION

Chemiluminescent technologies are widely utilized as detection reagents in clinical diagnostic platforms. Direct chemiluminescent labels, such as acridinium esters or isoluminol, provide shorter detection times due to the flash chemiluminescent signal being generated immediately after addition of triggering solutions. Enzyme-linked chemiluminescent substrates, such as 1,2-dioxetanes or luminol, have longer incubation times after their addition, but also provide ultrasensitive detection and greater substrate reagent stability. There is a need for enzyme substrates which provide a flash chemiluminescent signal and/or ultrasensitive detection and greater substrate reagent stability.

US 2006 0079699 A1 discloses methods for synthesizing 1,2-dioxetane compounds, including the compound CDP Star®, and intermediate compounds useful in the synthesis of these dioxetane compounds.

Product information leaflet "CSPD, ready-to-use" (Version 5, May 2003, Roche Diagnostics Corporation) describes the compound disodium 3-(4-methoxyspiro{1,2-dioxetane-3,2'-(5'-chloro)tricyclo[3.3.1.1^{3,7}]decan}-4-yl)phenyl phosphate (Cat. No. 1 755 633).

### SUMMARY OF THE INVENTION

In one aspect, the present invention provides compounds having the structure 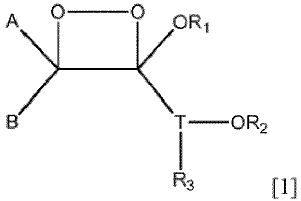 wherein, A and B arc independently selected from the group consisting of straight chain alkyl comprising 1 to 20 carbon atoms, straight chain alkenyl comprising 2 to 20 carbon atoms, branched alkyl comprising 3 to 20 carbon atoms, branched alkenyl comprising 3 to 20 carbon atoms, cycloalkyl comprising 3 to 20 carbon atoms, cycloalkenyl comprising 3 to 20 carbon atoms, cycloheteroalkyl comprising 3 to 20 carbon atoms, cycloheteroalkenyl comprising 3 to 20 carbon atoms, polycycloalkyl comprising 4 to 60 carbon atoms, polycycloalkenyl comprising 4 to 60 carbon atoms, polycycloheteroalkyl comprising 4 to 60 carbon atoms and polycycloheteroalkenyl comprising 4 to 60 carbon atoms, any one of which can be unsubstituted or substituted with one or more electron-active groups, solubilizing groups, or light-enhancing groups, and where A and B together form the cycloalkyl, cycloalkenyl, cycloheteroalkyl, cycloheteroalkenyl, polycycloalkenyl, polycycloheteroalkyl and polycycloheteroalkenyl, the formed group can be unsubstituted or substituted with one or more electron-active groups, solubilizing groups, or light-enhancing groups, or A and B together form the polycycloalkyl that is substituted with a halogen; 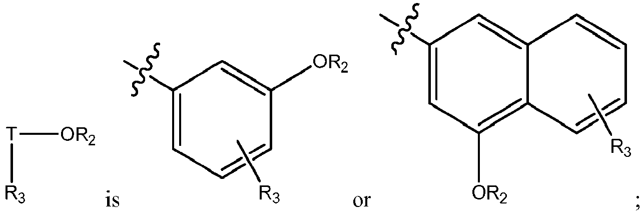 R₁ is straight chain alkyl comprising 1 to 20 carbon atoms which may be optionally substituted with one or more halogen atoms or branched chain alkyl comprising 3 to 20 carbon atoms which may be optionally substituted with one or more halogen atoms; R₂ is an enzyme-cleavable group that comprises a bond cleavable by an enzyme moiety to yield an oxygen anion on T; and R₃ is hydrogen or an electron-donating group; with the proviso that when R₁ is unsubstituted, R₃ is an electron-donating group; wherein, when 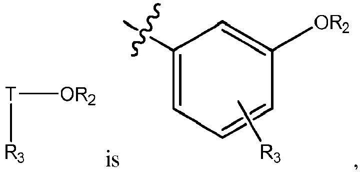 it is 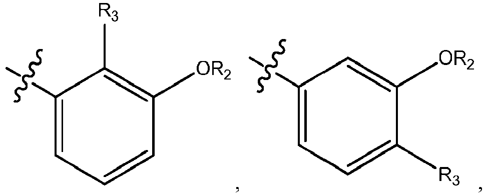 or 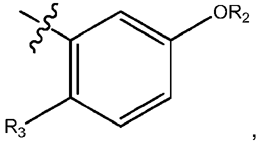 as further defined by the appended claims.

In some embodiments, the cleavage of the bond cleavable by an enzyme moiety results in the generation of light at 25°C which may reach a maximum in less than about 15 minutes. In some of these embodiments, the generation of light may reach a maximum in less than about 10 minutes; while in others, the generation of light may reach a maximum in less than about 5 minutes.

In some embodiments, the cleavage of the bond cleavable by an enzyme moiety results in the generation of light at 37°C which may reach a maximum in less than about 15 minutes. In some of these embodiments, the generation of light may reach a maximum in less than about 10 minutes; while in others, the generation of light may reach a maximum in less than about 5 minutes.

In some embodiments, the compound may be 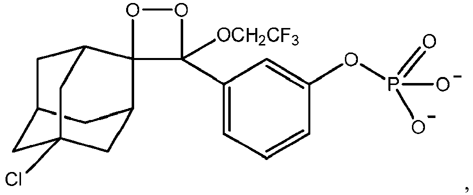 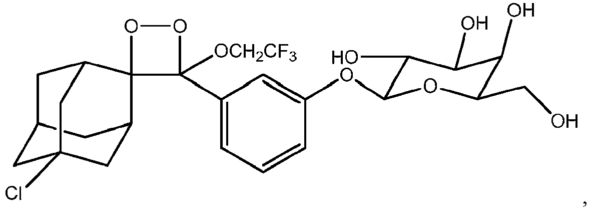 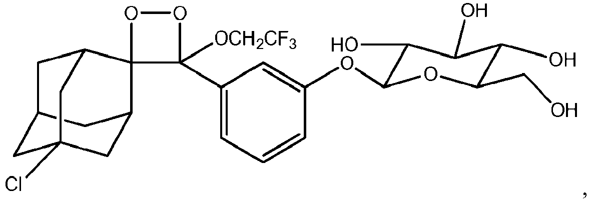 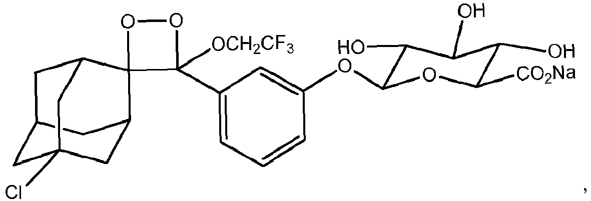 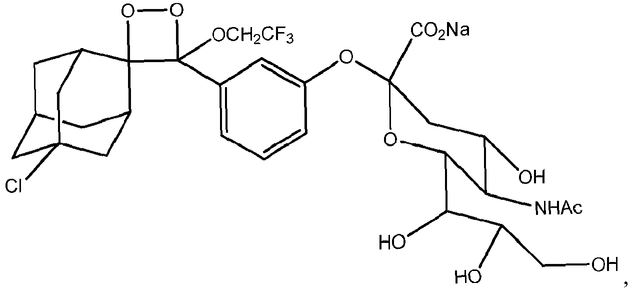 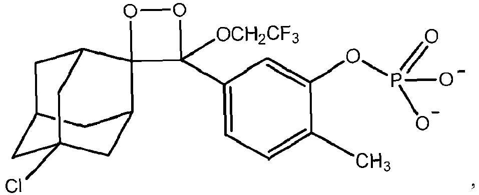 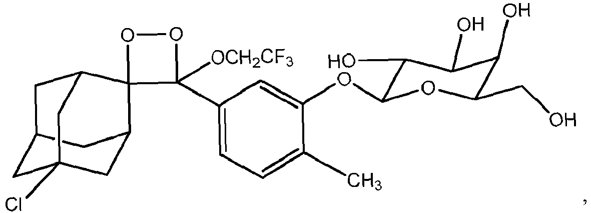 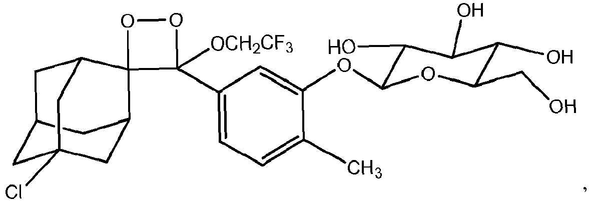 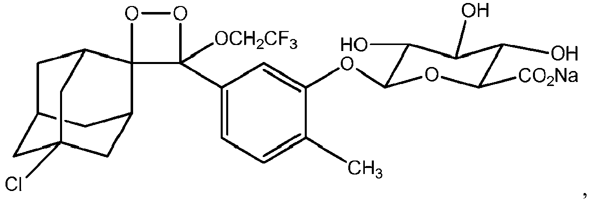 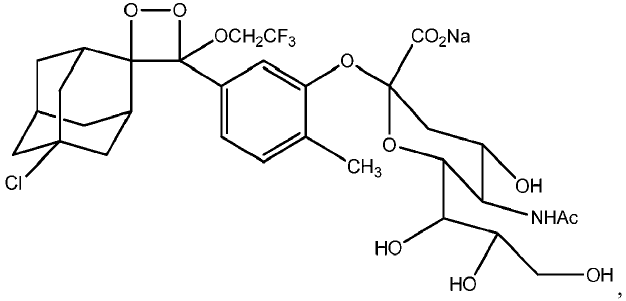 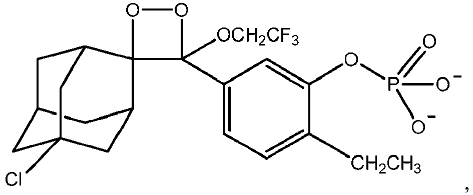 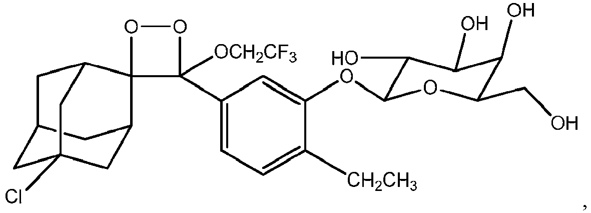 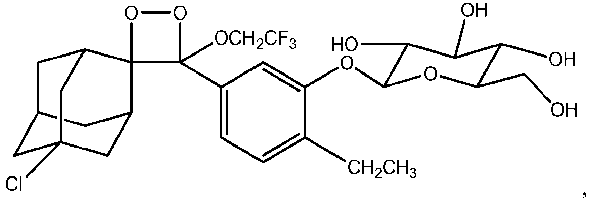 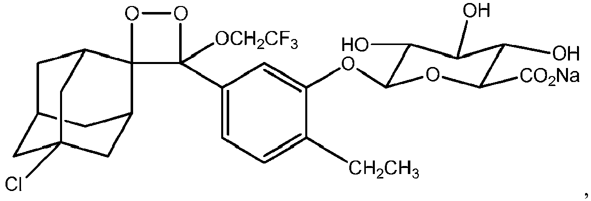 or

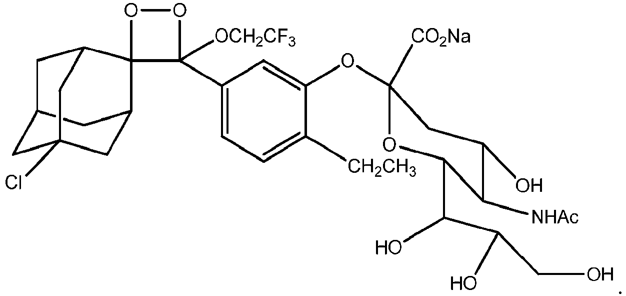

In another aspect, the present invention provides a method for generating light, comprising the steps of providing the compound [1]; providing an enzyme complex comprising an enzyme moiety which is capable of cleaving the compound; contacting the enzyme complex with the compound to form a reaction mixture; and, allowing the reaction mixture to generate light.

In some embodiments, the generation of light at 25°C may reach a maximum in less than about 15 minutes. In some these embodiments, the generation of light may reach a maximum in less than about 10 minutes; while in others, the generation of light may reach a maximum in less than about 5 minutes.

In some embodiments, the generation of light at 37°C may reach a maximum in less than about 15 minutes. In some of these embodiments, the generation of light may reach a maximum in less than about 10 minutes; while in others, the generation of light may reach a maximum in less than about 5 minutes.

In some embodiments, the enzyme moiety may comprise a hydrolytic enzyme. In some of these embodiments, the hydrolyic enzyme may be alkaline phosphatase, β-galactosidase, β-glucosidase, β-glucuronidase, or neuraminidase.

In some embodiments, the enzyme moiety may be an enzyme.

In some of these embodiments, the method may further comprise the step of detecting the light generated by the reaction mixture after addition of the compound, wherein the generation of light is indicative of the presence of the enzyme and the amount of light generated can be correlated to the amount of the enzyme present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some embodiments, the enzyme moiety may be an enzyme-linked antibody comprising a first antibody capable of binding to an antigen and an enzyme capable of cleaving the compound so that the substrate decomposes and generates light.

In some of these embodiments, the first antibody which may be covalently or non-covalently linked to the enzyme. In some of these embodiments, the first antibody may be covalently linked to a label and the enzyme is covalently linked to a molecule capable of non-covalent binding to the label. In some of these embodiments, the label may be biotin, or a biotin derivative, and the molecule may be avidin or strepavidin; while in others, the label may be a hapten and the molecule may be an antibody capable of binding to the hapten.

In some of these embodiments, the method may further comprise the steps of providing a sample suspected of comprising an antigen; providing a solid phase comprising a second antibody capable of binding to the antigen; contacting the sample and enzyme-linked antibody with the solid phase to form the enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some of these embodiments, the method may further comprise the step of removing any unbound enzyme-linked antibody from the enzyme complex.

In some embodiments, the enzyme moiety may be an enzyme-linked antigen comprising an antigen and an enzyme capable of cleaving the compound so that it decomposes and generates light.

In some of these embodiments, the antigen may be covalently or non-covalently linked to the enzyme. In some of these embodiments, the antigen may be covalently linked to a label and the enzyme is covalently linked to a molecule capable of non-covalent binding to the label. In some of these embodiments the label may be biotin, or a biotin derivative, and the molecule is avidin or strepavidin; while in others, the label may be a hapten and the molecule is an antibody capable of binding to the hapten.

In some of the embodiments, the method may further comprise the steps of providing a sample suspected of comprising an antigen; providing a solid phase comprising an antibody capable of binding to the antigen; contacting the sample and enzyme-linked antigen with the solid phase to form an enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some of these embodiments, the method may further comprise the step of removing any unbound enzyme-linked antigen from the enzyme complex.

In some embodiments, the enzyme moiety may be an enzyme-linked oligonucleotide comprising an oligonucleotide capable of hydridizing to a nucleic acid and an enzyme capable of cleaving the compound so that it decomposes and generates light.

In some of these embodiments, the oligonucleotide may be covalently or non-covalently linked to the enzyme. In some of these embodiments, the oligonucleotide may be covalently linked to a label and the enzyme is covalently linked to a molecule capable of non-covalent binding to the label. In some of these embodiments, the label may be biotin, or a biotin derivative, and the molecule is avidin or strepavidin; while in others, the label may be a hapten and the molecule is an antibody capable of binding to the hapten.

In some of these embodiments, the method may further comprise the steps of providing a sample suspected of comprising a nucleic acid; immobilizing the nucleic acid to a solid phase, contacting the immobilized nucleic acid and the enzyme-linked oligonucleotide to form an enzyme complex; and, detecting the light generated by the reaction mixture after addition of the compound, wherein the generation of light is indicative of the presence of the nucleic acid and the amount of light generated can be correlated to the amount of the nucleic acid present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes

In some of these embodiments, the method may further comprise the step of removing any unbound enzyme-linked oligonucleotide from the enzyme complex.

In some embodiments, the reaction mixture may further comprise an enhancer. In some these embodiments, the enhancer may comprise a polymeric quaternary ammonium salt, polymeric quaternary phosphonium salt, or a combination thereof. In some of these embodiments, the polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)], or a combination thereof; while in others, the polymeric quaternary phosphonium salt may be poly(vinylbenzyltrimethylphosphonium chloride), poly(vinylbenzyltributylphosphonium chloride), poly(vinylbenzyltrioctylphosphonium chloride), a copolymer comprising poly(vinylbenzyltributylphosphonium chloride) and poly(vinylbenzyltrioctylphosphonium chloride), or a combination thereof.

In some of these embodiments, the enhancer may further comprise an acceptor dye. In some of these embodiments, the acceptor dye may be fluorescein, rhodamine, sulforhodamine, ALEXA FLUOR 350, ALEXA FLUOR 405, ALEXA FLUOR 430, ALEXA FLUOR 488, ALEXA FLUOR 532, ALEXA FLUOR 546, or ALEXA FLUOR 555.

In some embodiments, the compound may be 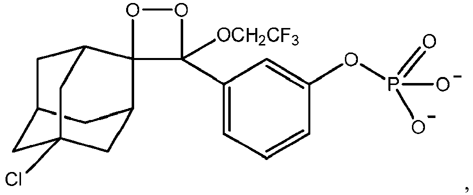 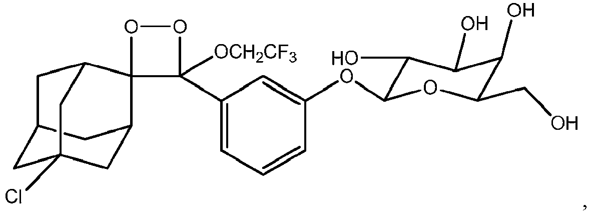 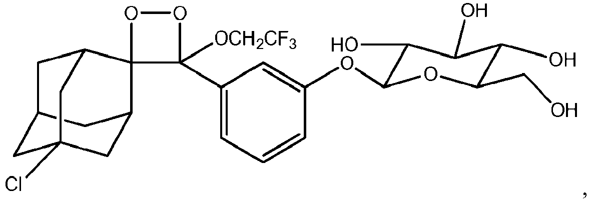 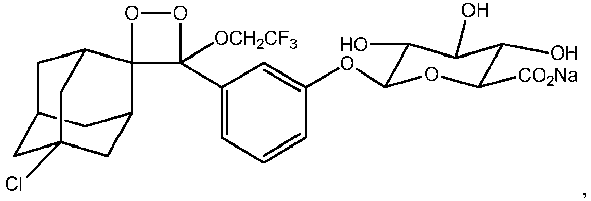 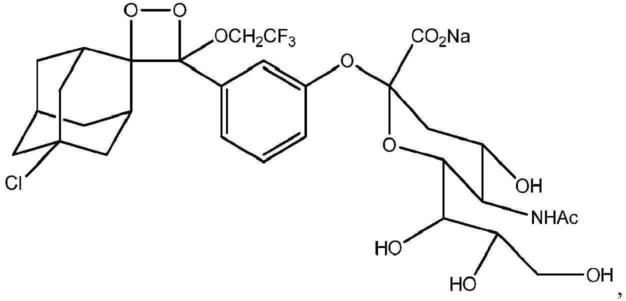 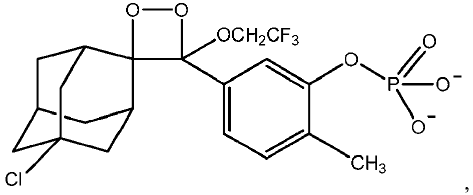 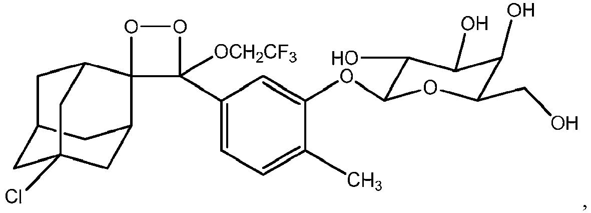 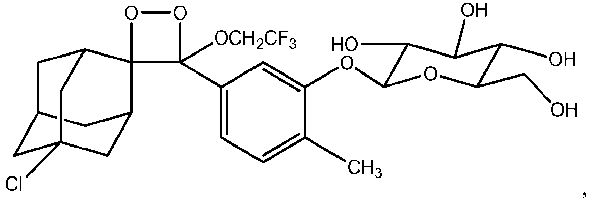 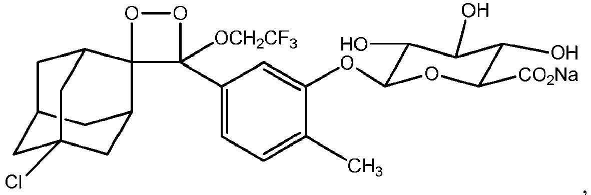 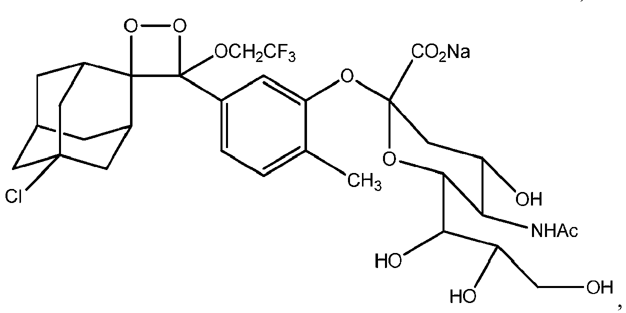 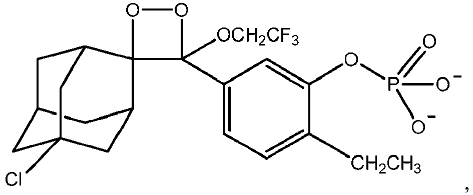 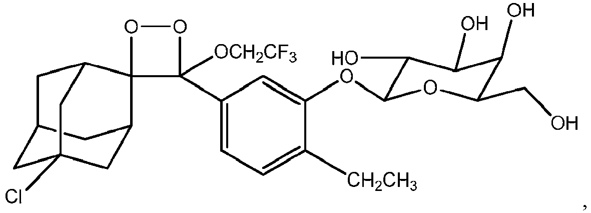 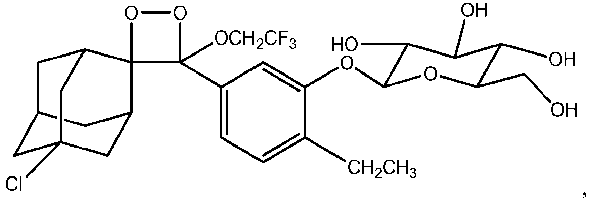 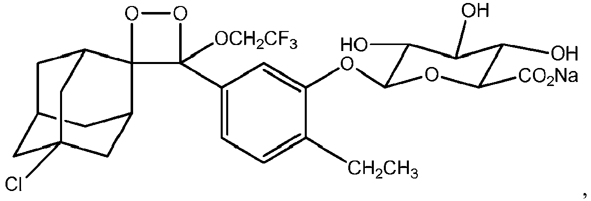 or

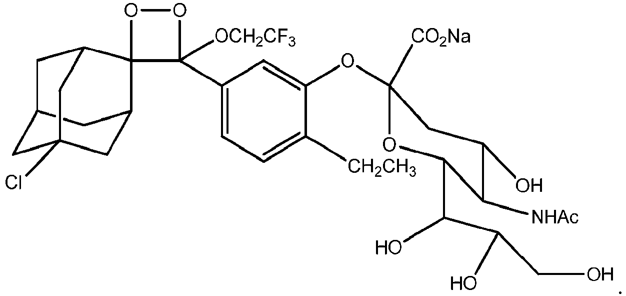

In another aspect, the present invention provides an assay method for determining the presence and/or amount of an enzyme in a sample, comprising the steps of providing the compound [1]; providing a sample suspected of comprising the enzyme which is capable of cleaving the compound so that it decomposes and generates light; contacting the sample with the compound to form a reaction mixture; and, detecting the light generated by the reaction mixture after addition of the compound, wherein the generation of light is indicative of the presence of the enzyme and the amount of light generated can be correlated to the amount of the enzyme present in the sample, and wherein the generation of light at 25°C to 37°C reaches a maximum in less than about 15 minutes.

In some embodiments, the generation of light may reach a maximum in less than about 10 minutes; while in others, the generation of light may reach a maximum in less than about 5 minutes.

In another aspect, the present invention provides an assay method for determining the presence and/or amount of an antigen in a sample, comprising the steps of providing the compound [1]; providing a sample suspected of comprising the antigen; providing an enzyme-linked antigen comprising the antigen and an enzyme capable of cleaving the compound so that it decomposes and generates light; providing a solid phase comprising an antibody capable of binding to the antigen; contacting the sample and enzyme-linked antigen with the solid phase to form an enzyme complex; contacting the enzyme complex with the compound to form a reaction mixture; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes.

In some embodiments, the generation of light may reach a maximum in less than about 10 minutes; while in others, the generation of light may reach a maximum in less than about 5 minutes.

In another aspect, the present invention provides an assay method for determining the presence and/or amount of a nucleic acid in a sample, comprising the steps of providing the compound [1]; providing a sample suspected of comprising the nucleic acid; immobilizing the nucleic acid to a solid phase; providing an enzyme-linked oligonucleotide comprising an oligonucleotide capable of hydridizing to the nucleic acid and an enzyme capable of cleaving the compound so that it decomposes and generates light; contacting the immobilized nucleic acid and enzyme-linked oligonucleotide to form an enzyme complex; contacting the enzyme complex with the compound to form a reaction mixture; and, detecting the light generated by the reaction mixture after addition of the compound, wherein the generation of light is indicative of the presence of the nucleic acid and the amount of light generated can be correlated to the amount of the nucleic acid present in the sample, and wherein the generation of light at 25°C to 37°C reaches a maximum in less than about 15 minutes.

In some embodiments, the generation of light may reach a maximum in less than about 10 minutes; while in others, the generation of light may reach a maximum in less than about 5 minutes.

In another aspect, the present invention provides a kit for detecting the presence and/or amount of an analyte in a sample comprising the compound [1]; and a buffer.

In some embodiments, the kit may further comprise an enhancer. In some of these embodiments, the enhancer may comprise a polymeric quaternary ammonium salt, polymeric quaternary phosphium salt, or a combination thereof. In some of these embodiments, the polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)], or a combination thereof; while others, the the polymeric quaternary phosphonium salt may be poly(vinylbenzyltrimethylphosphonium chloride), poly(vinylbenzyltributylphosphonium chloride), poly(vinylbenzyltrioctylphosphonium chloride), a copolymer comprising poly(vinylbenzyltributylphosphonium chloride) and poly(vinylbenzyltrioctylphosphonium chloride); or a combination thereof.

In some embodiments, the enhancer may further comprise an acceptor dye. In some of these embodiments, the acceptor dye may be fluorescein, rhodamine, sulforhodamine, ALEXA FLUOR 350, ALEXA FLUOR 405, ALEXA FLUOR 430, ALEXA FLUOR 488, ALEXA FLUOR 532, ALEXA FLUOR 546, or ALEXA FLUOR 555.

In some embodiments, the cleavage of the bond of the compound cleavable by an enzyme moiety results in the generation of light at 25°C which may reach a maximum in less than about 15 minutes. In some of these embodiments, the generation of light may reach a maximum in less than about 10 minutes; while others, the generation of light may reach a maximum in less than about 5 minutes.

In some embodiments, the cleavage of the bond of the compound cleavable by an enzyme moiety results in the generation of light at 37°C which may reach a maximum in less than about 15 minutes. In some of these, the generation of light may reach a maximum in less than about 10 minutes; while in others, the generation of light may reach a maximum in less than about 5 minutes.

In some embodiments, the compound may be 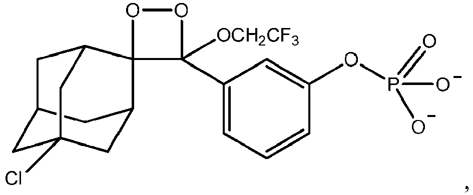 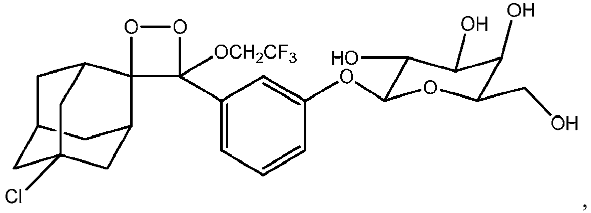 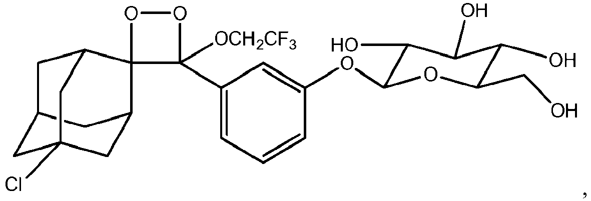 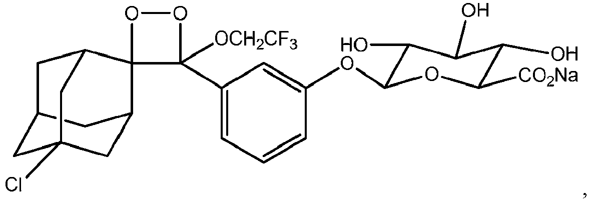 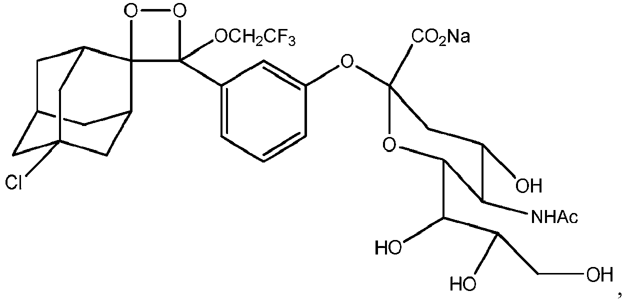 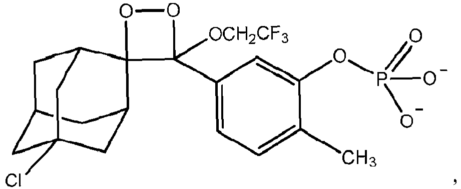 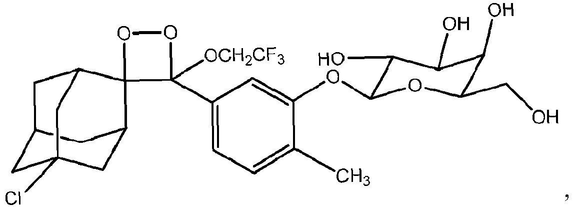 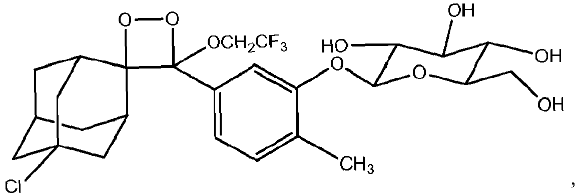 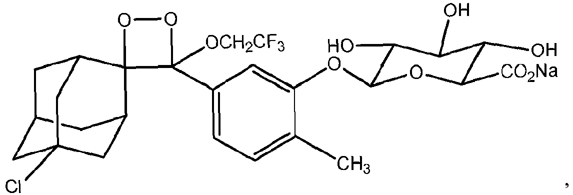 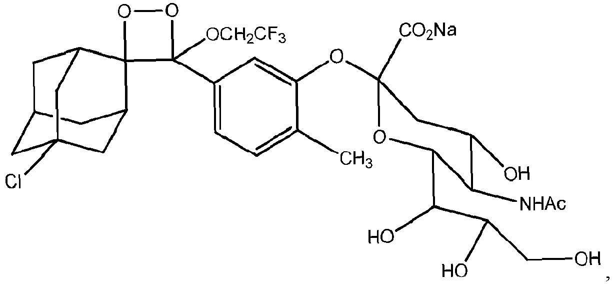 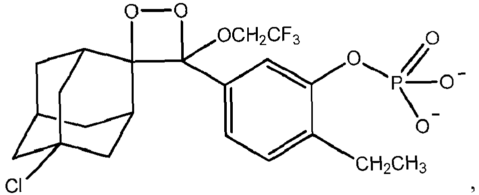 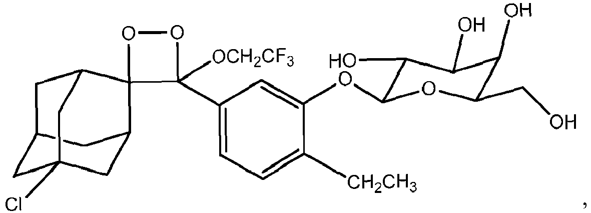 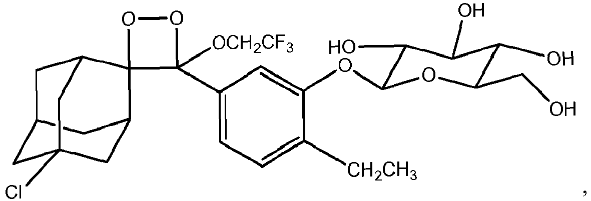 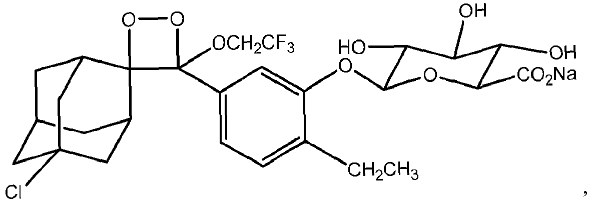 or

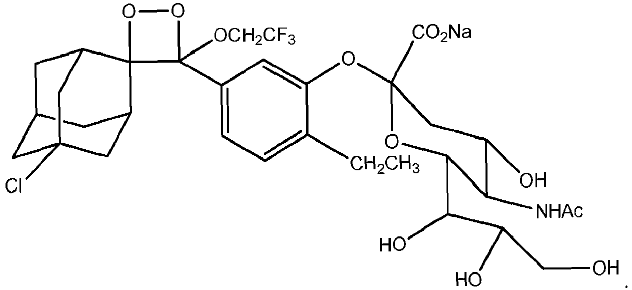

In another aspect, the presentdisclosure provides a method for generating light, comprising the steps of providing the compound having the structure 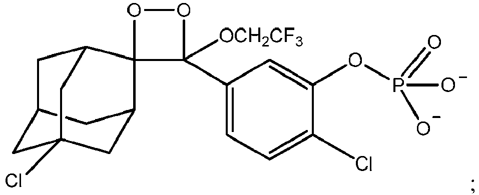 providing an enzyme complex comprising an enzyme moiety which is capable of cleaving the compound; contacting the enzyme complex with the compound to form a reaction mixture; and, allowing the reaction mixture to generate light; provided that when the enzyme complex comprises a solid phase, an antigen is immobilized to the solid phase, and the antigen is protein, then the solid phase is not a membrane or microassay.

In some embodiments, the generation of light at 25°C to 37°C may reach a maximum in less than about 15 minutes. In some of these embodiments, the generation of light may reach a maximum in less than about 10 minutes; while in others, the generation of light may reach a maximum in less than about 5 minutes.

In some embodiments, the enzyme moiety may be alkaline phosphatase.

In some of these embodiments, the method may further comprise the step of detecting the light generated by the reaction mixture after addition of the compound, wherein the generation of light is indicative of the presence of the alkaline phosphatase and the amount of light generated can be correlated to the amount of the alkaline phosphatase present in the sample, and wherein the generation of light at 25° C to 37°C reaches a maximum in less than about 15 minutes. In some of these, the generation of light may reach a maximum in less than about 10 minutes; while in others, the generation of light may reach a maximum in less than about 5 minutes.

In some embodiments, the enzyme moiety may be an alkaline phosphatase-linked antibody comprising a first antibody capable of binding to an antigen.

In some of these embodiments, the first antibody may be covalently or non-covalently linked to the alkaline phosphatase. In some of these embodiments, the first antibody may be covalently linked to a label and the alkaline phosphatase may be covalently linked to a molecule capable of non-covalent binding to the label. In some of these embodiments, the label may be biotin, or a biotin derivative, and the molecule may be avidin or strepavidin; while in others, the label may be a hapten and the molecule may be an antibody capable of binding to the hapten.

In some of these embodiments, the method may further comprise the steps of providing a sample suspected of comprising an antigen; providing a solid phase comprising a second antibody capable of binding to the antigen; contacting the sample and alkaline phosphatase-linked antibody with the solid phase to form the enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some of these embodiments, the method may further comprise the step of removing any unbound alkaline phosphatase-linked antibody from the enzyme complex.

In some embodiments, the enzyme moiety may be an alkaline phosphatase-linked antigen comprising an antigen.

In some of these embodiments, the antigen may be covalently or non-covalently linked to the alkaline phosphatase. In some of these embodiments, the antigen may be covalently linked to a label and the alkaline phosphatase may be covalently linked to a molecule capable of non-covalent binding to the label. In some of these embodiments, the label may be biotin, or a biotin derivative, and the molecule may be avidin or strepavidin; while in others, the label may be a hapten and the molecule may be an antibody capable of binding to the hapten.

In some of these embodiments, the method may further comprise the steps of providing a sample suspected of comprising an antigen; providing a solid phase comprising an antibody capable of binding to the antigen; contacting the sample and alkaline phosphatase-linked antigen with the solid phase to form an enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some of these embodiments, the method may further comprise the step of removing any unbound alkaline phosphatase-linked antigen from the enzyme complex.

In some embodiments, the enzyme moiety may be an alkaline phosphatase-linked oligonucleotide comprising an oligonucleotide capable of hydridizing to a nucleic acid.

In some of these embodiments, the oligonucleotide may be covalently or non-covalently linked to the alkaline phosphatase. In some of these embodiments, the oligonucleotide may be covalently linked to a label and the alkaline phosphatase may be covalently linked to a molecule capable of non-covalent binding to the label. In some of these embodiments, the label may be biotin, or a biotin derivative and the molecule may be avidin or strepavidin, while in others, the label may be a hapten and the molecule may be an antibody capable of binding to the hapten.

In some of these embodiments, the may further comprise the steps of providing a sample suspected of comprising a nucleic acid; immobilizing the nucleic acid to a solid phase; contacting the immobilized nucleic acid and the alkaline phosphatase-linked oligonucleotide to form an enzyme complex; and, detecting the light generated by the reaction mixture after addition of the compound, wherein the generation of light is indicative of the presence of the nucleic acid and the amount of light generated can be correlated to the amount of the nucleic acid present in the sample, and wherein the generation of light at 25°C to 37°C reaches a maximum in less than about 15 minutes. In some of these embodiments, the generation of light may reach a maximum in less than about 10 minutes; while in others, the generation of light may reach a maximum in less than about 5 minutes.

In some of these embodiments, the method may further comprise the step of removing any unbound enzyme-linked oligonucleotide from the enzyme complex.

In some embodiments, the reaction mixture may further comprise an enhancer.

In some of these embodiments, the enhancer may comprise a polymeric quaternary ammonium salt, polymeric quaternary phosphonium salt, or a combination thereof. In some of these embodiments, the polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)], or a combination thereof; while in others, the polymeric quaternary phosphonium salt is poly(vinylbenzyltrimethylphosphonium chloride), poly(vinylbenzyltributylphosphonium chloride), poly(vinylbenzyltrioctylphosphonium chloride), a copolymer comprising poly(vinylbenzyltributylphosphonium chloride) and poly(vinylbenzyltrioctylphosphonium chloride), or a combination thereof.

In some of these embodiments, the enhancer may further comprise an acceptor dye. In some of these embodiments, the acceptor dye may be fluorescein, rhodamine, sulforhodamine, ALEXA FLUOR 350, ALEXA FLUOR 405, ALEXA FLUOR 430, ALEXA FLUOR 488, ALEXA FLUOR 532, ALEXA FLUOR 546, or ALEXA FLUOR 555.

### DESCRIPTION OF THE DRAWINGS

FIG. 1 shows the two-step synthesis of 4-Me-3-OBn-benzaldehyde.
FIG. 2 shows the two-step, one pot synthesis of 4-Me-Phe phosphonate.
FIG. 3 shows two-step synthesis of 4-Me-Phe enol ether by Horner Emmons coupling followed by debenzylation.
FIG. 4 shows two-step synthesis of 4-Me-Phe dioxetane starting with 4-Me-Phe enol ether.

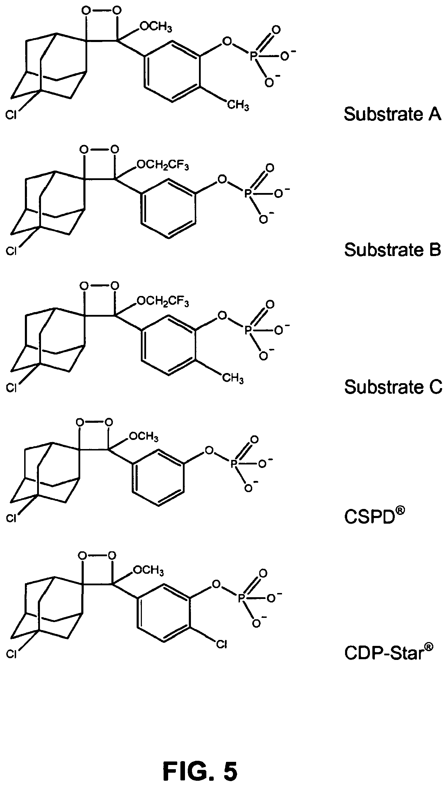

FIG. 5 shows the structures of the flash substrates of the present invention, Substrate A, Substrate B and Substrate C, along with those of commercially available CSPD® and CDP-Star® (both from Life Technologies, San Diego, California).
FIG. 6 shows the results of a kinetic evaluation of Substrates A and B, CSPD and CDP-Star performed at 25°C.
FIG. 7 shows purified alkaline phosphatase sensitivity curves performed at 25 ° C. Sensitivity is defined as the concentration of alkaline phosphatase (AP) where the signal to-noise ratio is 2.
FIG. 8 shows a kinetic evaluation of Substrate A, CSDP, and CDP-Star in an IL-6 immunoassay performed at 25°C.
FIG. 9 shows the signal-to-noise ratios at various time points at 25°C for CDP-
   Star/Sapphire-II and Substrate A.
FIG. 10 shows a kinetic evaluation of Substrates A, B and C in an IL-6 immunoassay performed at 37°C.
FIG. 11 shows a comparison of signal versus signal-to-noise kinetics for Substrates A, B, C and a competitor's substrate at 37°C in an immunoassay system.
FIG. 12 shows a comparison of sensitivity in an IL-6 immunoassay system.
FIG. 13 shows the results of shelf life stability study for Substrate A.
FIG. 14 shows the results of a kinetic evaluation of Substrates A, B and C, CDP-Star/Emerald-II, Competitor's Flash Substrate D, and Competitor's Glow Substrate E, with strepavidin-labeled Dynabeads M-280 Tosylactivated bound with biotin-labeled alkaline phosphatase.
FIG. 15 shows the sensitivity curves of strepavidin-labeled Dynabeads M-280 Tosylactivated bound with variable amounts of biotin-labeled alkaline phosphatase at optimum read time and at room temperature.
FIG. 16 shows the sensitivity curves of strepavidin-labeled Dynabeads M-280 Tosylactivated bound with variable amounts of biotin-labeled alkaline phosphatase at optimum read time, at room temperature, where the signal is read at 2 minutes.
FIG. 17 shows the light emission kinetics versus signal-to-noise measured at 37 ° using Dynabeads MyOne Carboxylic Acid and Substrate A/Emerald-III, Substrate B, and CDP-Star/Emerald II.
FIG. 18 shows the performance in an human cardiac troponin (cTnl) immunoassay performed at 37°C on Dynabeads MyOne Carboxylic Acid beads coupled with anti-cTnl using Substrate A/Emerald-III, Substrate B, and CDP-Star/Emerald-II.
FIG. 19 shows the cTnl sensitivity curves measured at 37°C for the cTnl immunoassays based on Dynabeads MyOne Carboxylic Acid, Dynabeads M-280, Dynabeads M-270 beads using Substrate A.
FIG. 20 shows the performance of the cTnl immunoassay based on Dynabeads M-270 epoxy beads with Substrates A, B and C, CDP-Star/Emerald-II, and Vendor X's Substrate D at their respective read times.

### DETAILED DESCRIPTION OF THE INVENTION

Before describing the present invention in detail, it is to be understood that this invention is not limited to the embodiments disclosed herein. Further, while the methods and kits are described in terms of "comprising" various steps or components (interpreted as meaning, "including, but not limited to"), the methods and kits can also "consist essentially of" or "consist of" the various steps and components, such terminology should be interpreted as defining essentially closed-member groups. Finally, it is be noted that, as used in this specification and the appended claims, the singular form "a", "an" and "the" include plural referents unless the context clearly dictates otherwise.

### Definitions:

Acceptor dye refers to a molecule which can accept energy, especially light, from another light-emitting molecule and in turn emit detectable energy, again preferably light.

Analyte refers to a substance or chemical constituent that is determined in an analytical procedure. As used herein, the term includes, but is not limited to, an antigen or antibody.

Antigen refers to a substance to which an antibody can bind.

Antibody refers to gamma globulin proteins that are found in blood or other bodily fluids of vertebrates, and are used by the immune system to identify and neutralize foreign objects. As used herein, the term includes, but is not limited to, any polyclonal, monoclonal, recombinant antibody or fragments thereof which can bind an antigen.

Enhancer refers to a water-soluble substance that increases specific light energy production resulting from the enzymatic cleavage of a 1,2-dioxetane enzyme substrate and its subsequent decomposition, where this light production observed is above that observed in the absence of the enhancer.

Enzyme refers to proteins that catalyze chemical reactions.

Hapten refers to a small molecule that can elicit an immune response only when attached to a large carrier such as a protein.

Hydrolytic enzyme or hydrolase refers to an enzyme that catalyzes the hydrolysis of a chemical bond and would be classified as EC 3 in the EC number classification of enzymes.

Nucleic acid refers to single-stranded or double-stranded DNA, RNA or fragment thereof.

Oligonucleotide refers to a short nucleic acid polymer. As used here, the term includes, but is not limited, to a nucleic acid polymer comprising 2 to 1000 nucleic acids.

The invention provides compounds which are 1,2-dioxetanes. The invention also provides methods of using one or more of the compounds of the present invention. The methods provided are for generating a light. The generation of light can be used to determine the presence and/or amount in a sample of one or more analytes, which include an enzyme, antigen, or a nucleic acid. The present invention also provides kits for detection of the presence and/or amount of one or more analytes in a sample. In another aspect, the invention provides kits for generating light. The kits comprise one or more of compounds of the present invention. These compounds, methods and the kits, have utility in art-recognized assays.

In one aspect, the present invention provides compounds which may be 1,2-dioxetanes. These are defined in the claims, and include those having the structures [1] with the respective substituents as described below 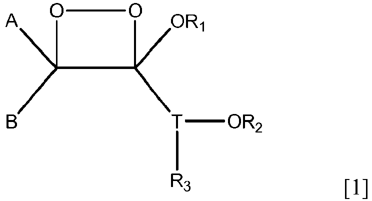 wherein,
A and B may be independently selected from the group consisting of straight chain alkyl, straight chain alkenyl, branched alkyl, branched alkenyl, cycloalkyl, cycloalkenyl, cycloheteroalkyl, cycloheteroalkenyl, polycycloalkyl, polycycloalkenyl, polycycloheteroalkyl and polycycloheteroalkenyl, any one of which can be unsubstituted or substituted with one or more electron-active groups, solubilizing groups, or light-enhancing groups, and where A and B together form the cycloalkyl, cycloalkenyl, cycloheteroalkyl, cycloheteroalkenyl, polycycloalkenyl, polycycloheteroalkyl and polycycloheteroalkenyl, the formed group can be unsubstituted or substituted with one or more electron-active groups, solubilizing groups, or light-enhancing groups, or A and B together form the polycycloalkyl that is substituted with a halogen; 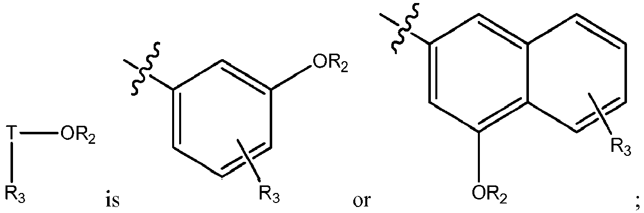
   R₁ may be a straight chain alkyl comprising 1 to 20 carbon atoms which may be optionally substituted with one or more halogen atoms or branched chain alkyl comprising 3 to 20 carbon atoms which may be optionally substituted with one or more halogen atoms;
   R₂ may be an enzyme-cleavable group that comprises a bond cleavable by an enzyme moiety to yield an oxygen anion on T; and
   R₃ may be hydrogen or an electron-donating group; with the proviso that when R₁ is unsubstituted, R₃ is an electron-donating group;
      wherein, when 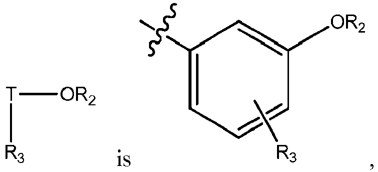 it may be 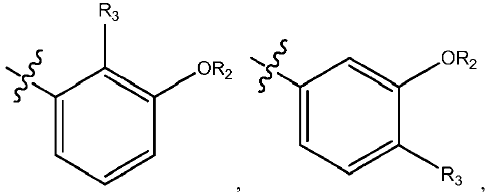 or 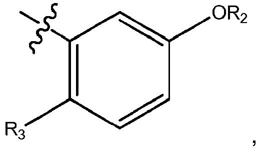 as further defined by the appended claims.

In some embodiments of compounds [1], R₂, the enzymatically cleavable substituent, may be a phosphate ester group represented by the general formula: 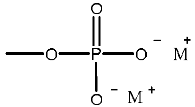 wherein M+ represents a cation such as alkali metal, e.g., sodium or potassium, ammonium, or a C₁₋₇ alkyl, aralkyl or aromatic quaternary ammonium cation, N(DR₃)₄ + in which each D₃ can be alkyl, e.g., methyl or ethyl, aralkyl, e.g., benzyl, or form part of a heterocyclic ring system, e.g., pyridinium, and particularly the disodium salt. Such quaternary ammonium cations can also be connected through one of their quaternizing groups to a polymeric backbone, viz. 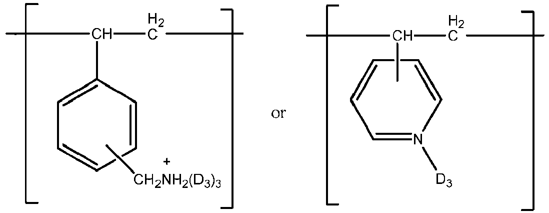 where n is greater than 1, or can be part of a polyquaternary ammonium salt, i.e., an ionene polymer.

In some embodiments of compounds [1] the cleavage of the bond cleavable by an enzyme moiety may result in the generation of light at 25 ° C which reaches a maximum in less than about 15 minutes. In other embodiments, the generation of light may reach a maximum in less than about 10 minutes. In other embodiments, the generation of light may reach a maximum in less than about 5 minutes.

In some embodiments of compounds [1] the cleavage of the bond cleavable by an enzyme moiety may result in the generation of light at 37°C which reaches a maximum in less than about 15 minutes. In other embodiments, the generation of light may reach a maximum in less than about 10 minutes. In other embodiments, the generation of light may reach a maximum in less than about 5 minutes.

In some embodiments, compound [1] may be: 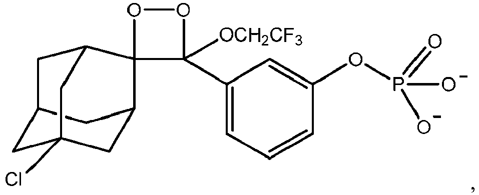 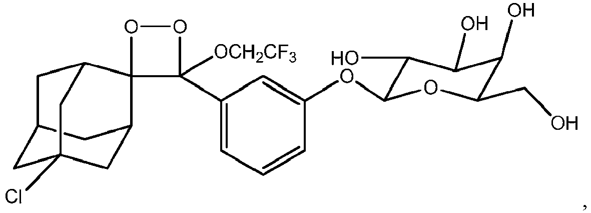 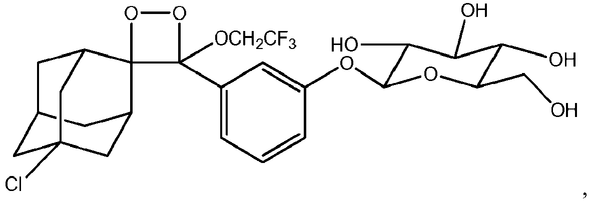 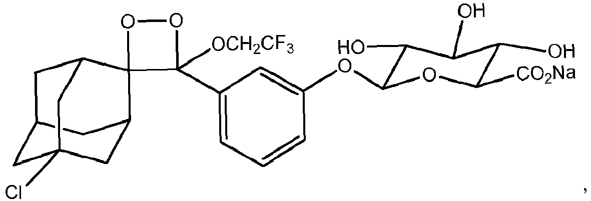 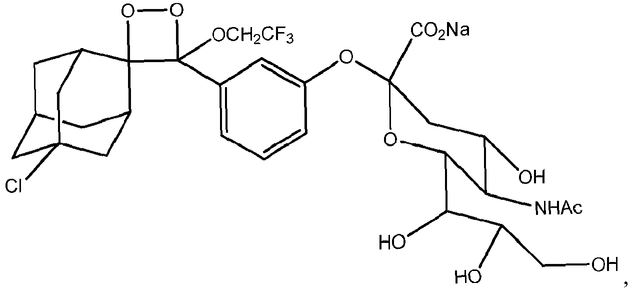 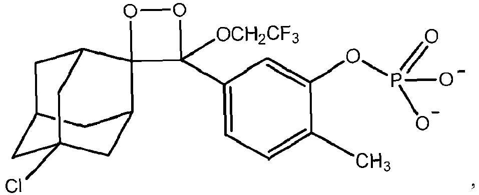 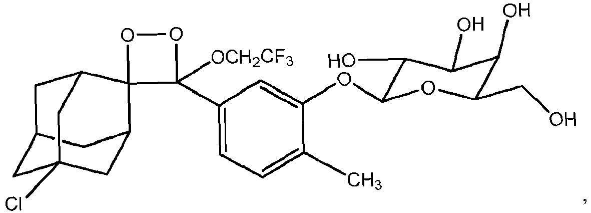 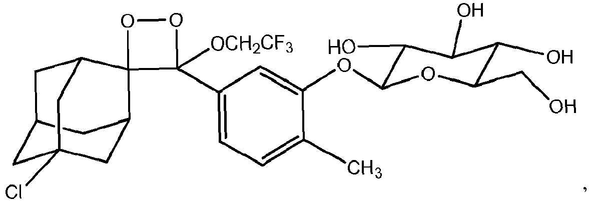 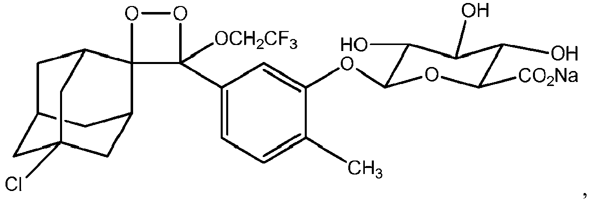 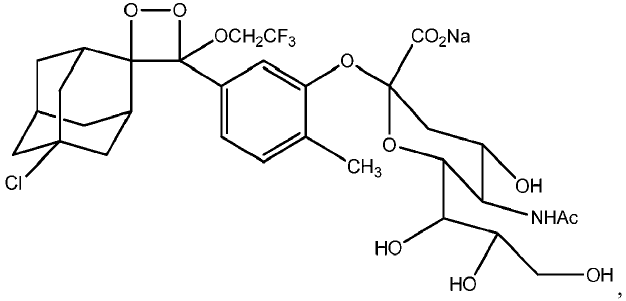 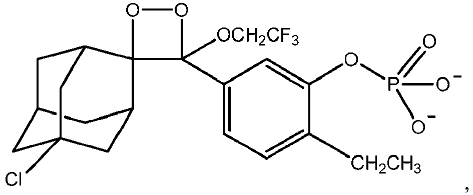 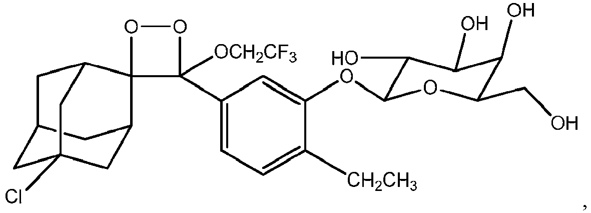 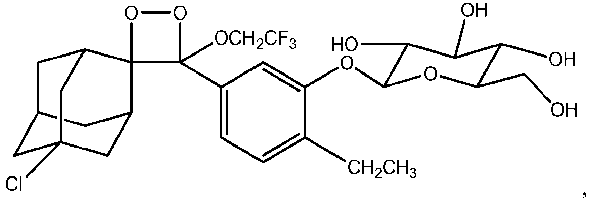 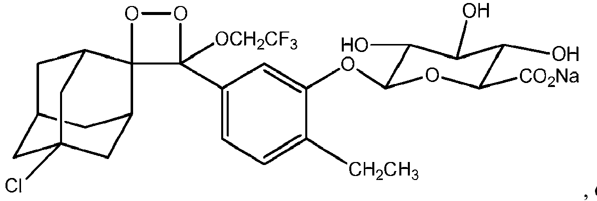 or

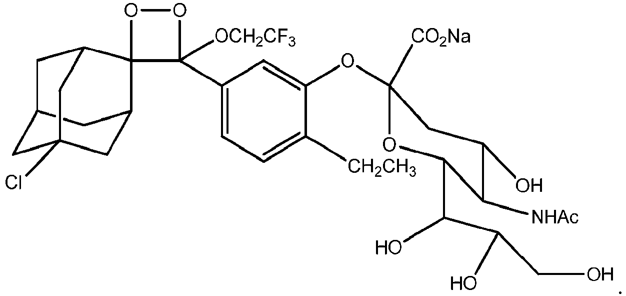

Any of the compounds of the present invention disclosed herein above can be used in the methods and kits disclosed below or in the above Summary of Invention.

In another aspect, the present invention provides methods. The methods may comprise the steps of providing a compound of the present invention as defined above; providing an enzyme complex comprising an enzyme moiety which is capable of cleaving the compound; contacting the enzyme complex with the compound to form a reaction mixture; and, allowing the reaction mixture to generate light.

In one step of these methods, a compound of the present invention is provided. Such a compound may have the formula [1] and may include any embodiment thereof as disclosed above or in the Summary of Invention.

In another step of these methods, a sample is provided which is suspected of comprising an enzyme, antigen, antibody, nucleic acid or analyte. Samples may be assayed which arc suspected of comprising an enzyme, antigen, nucleic acid or analyte. The sample may be of a biological or non-biological origin. Where the sample is of a biological origin, it may be blood, serum, plasma, urine, feces, saliva, mucus, seminal fluid, tissue, a tissue extract, cell culture media, cells, cell extracts, and the like.

In another step of these methods, an enzyme complex is provided which may be comprised of an enzyme moiety which is capable of cleaving the compound of the present invention as defined above. The enzyme moiety may be an enzyme, enzyme-linked antibody, enzyme-linked antigen or enzyme-linked oligonucleotide. The enzyme moiety may comprise an enzyme which is capable of cleaving a compound of the present invention. In some embodiments, the enzyme may be a hydrolytic enzyme. A hydrolytic enzyme includes enzymes which cleave ester bonds and is classified as EC 3.1 or cleave sugar bonds and is classified as EC 3.2, and includes, but is not limited to, alkaline phosphatase, β-galactosidase, β-glucosidase, β-glucuronidase or neuraminidase.

In some embodiments, the enzyme moiety may be an enzyme. In these embodiments, the enzyme in a sample comprises the enzyme complex. The enzyme complex is contacted with the compound of the present invention to form a reaction mixture and the reaction mixture is allowed to generate light.

In some embodiments, the emission of light may be detected and such emission will be indicative of the presence of the enzyme, and the amount of light emitted can be correlated to the amount of the enzyme present in the sample.

In some embodiments, the enzyme moiety may be an enzyme-linked antibody. The enzyme-linked antibody comprises a first antibody capable of binding to an antigen and an enzyme capable of cleaving the compound of the present invention so that the substrate decomposes and generates light. In these embodiments, the enzyme-linked antibody, antigen, and a second antibody which is capable of binding the antigen and immobilized on a solid phase comprises the enzyme complex. The enzyme complex is contacted with the compound of the present invention to form a reaction mixture and the reaction mixture is allowed to generate light.

In some embodiments, a sample suspected of comprising an antigen may be contacted with an enzyme-linked antibody comprising a first antibody and an enzyme and a solid phase comprising a second antibody, where both antibodies are capable of binding the antigen to provide an enzyme complex which is capable of cleaving the compound of the present invention so that the substrate decomposes and generates light. The sample, enzyme-linked antibody and solid phase may be combined in any order.

In some embodiments, the method may further comprise the step of removing any unbound enzyme-linked antibody from the enzyme complex by washing the enzyme complex. This may be performed by addition and removal of a buffer compatible with the components of the enzyme complex. Such buffers are well known in the diagnostic arts. Additional wash steps of the solid phase may be performed.

The first antibody, second antibody, or both may be a polyclonal, monoclonal or recombinant antibody.

The solid phase may be a bead, test tube, multi-well plate, microarray, gel, membrane, microparticles, nanocrystals, quantum dots and the like. The materials from which these solid phases are made are known in the diagnostic arts.

The second antibody may be immobilized on the solid phase by non-covalent or covalent attachment of the antibody to the solid phase, by techniques known in the diagnostic arts.

The first antibody may be linked to the enzyme covalently or non-covalently. When linked non-covalently, first antibody may be covalently linked to a label and the enzyme may be covalently linked to a molecule capable of non-covalent binding to the label.

In some embodiments, the label may be biotin, or biotin derivative, and the molecule may be avidin or strepavidin. A biotin derivative is a biotin molecule which has substitutions. A biotin molecule wherein a portion of biotin structure is missing is also considered a biotin derivative. Biotin derivatives include naturally-occurring biotin as well as synthetic biotin which are substituted.

In some embodiments, the label may be a hapten and the molecule may be an antibody capable of binding to the hapten. The use of digoxigenin as a hapten, and anti-digoxigenin as the molecule is known in the diagnostic arts.

In some embodiments, the enzyme moiety may be an enzyme-linked antigen. The enzyme-linked antigen comprises the antigen and an enzyme capable of cleaving the compound of the present invention. In these embodiments, the enzyme complex comprises the enzyme-linked antigen bound to solid phase comprising an antibody which is capable of binding the antigen. The enzyme complex is contacted with the compound of the present invention as defined above to form a reaction mixture and the reaction mixture is allowed to generate light.

In some embodiments, a sample suspected of comprising an antigen may contacted with the enzyme-linked antigen, and the solid phase comprising an antibody capable of binding to the antigen. The sample, enzyme-linked antigen and solid phase may be combined in any order.

In some embodiments, the methods may further comprise the step of removing any unbound enzyme-linked antigen from the enzyme complex by washing the enzyme complex. This may be performed by addition and removal of a buffer compatible with the components of the enzyme complex. Such buffers are well known in the diagnostic arts. Additional wash steps of the solid phase may be performed.

The antibody may be a polyclonal, monoclonal or recombinant antibody.

The solid phase may be a bead, test tube, multi-well plate, microarray, gel, membrane, microparticles, nanocrystals, quantum dots and the like. The materials from which these solid phases are made are known in the diagnostic arts.

The antibody may be immobilized to the solid phase by non-covalent or covalent attachment of the antibody to the solid phase.

The antigen may be linked to the enzyme covalently or non-covalently. When linked non-covalently, the antigen may covalently linked to a label and the enzyme may be covalently linked to a molecule capable of non-covalent binding to the label.

In some embodiments, the label may be biotin, or biotin derivative, as described above, and the molecule may be avidin or strepavidin.

In some embodiments, the label may be a hapten and the molecule may be an antibody capable of binding to the hapten. The use the digoxigenin as a hapten, and anti-digoxigenein as the molecule is known in the diagnostic arts.

In some embodiments, the enzyme moiety may be an enzyme-linked oligonucleotide. The enzyme-linked oligonucleotide comprises an oligonucleotide capable of hydridizing to certain nucleic acid and an enzyme capable of cleaving the compound of the present invention. In these embodiments, the enzyme complex comprises the enzyme-linked oligonucleotide hybridized to a solid phase comprising the nucleic acid. The enzyme complex is contacted with the compound of the present invention to form a reaction mixture and the reaction mixture is allowed to generate light.

In some embodiments, the methods may further comprise the steps of providing a sample suspected of comprising a nucleic acid; immobilizing the nucleic acid onto a solid phase; contacting the immobilized nucleic acid and the enzyme-linked oligonucleotide to form an enzyme complex; and, detecting the light emitted from the reaction mixture after addition of the aqueous solution of the 1,2-dioxetane enzyme substrate, wherein the emission of light is indicative of the presence of the nucleic acid, and the amount of light emitted can be correlated to the amount of the nucleic acid present in the sample.

In some embodiments, the method may further comprise the step of removing any unbound enzyme-linked oligonucleotide from the enzyme complex by washing the enzyme complex. This may be performed by addition and removal of a buffer compatible with the components of the enzyme complex. Such buffers are well known in the diagnostic arts. Additional washes of the solid phase may be performed.

The solid phase may be a bead, test tube, multi-well plate, microarray, gel, membrane, microparticles, nanocrystals, quantum dots and the like. The materials from which these are made are known in the diagnostic arts.

The oligonucleotide may be linked to the enzyme covalently or non-covalently. When linked non-covalently, the oligonucleotide may be covalently linked to a label and the enzyme may be covalently linked to a molecule capable of non-covalent binding to the label.

In some embodiments, the label may be biotin, or biotin derivative, as described above, and the molecule may be avidin or strepavidin.

In some embodiments, the label may be a hapten and the molecule may be an antibody capable of binding to the hapten. The use the digoxigenin as a hapten, and anti-digoxigenein as the molecule is known in the diagnostic arts.

In another step of this method, the sample, either alone or as a complex comprising an enzyme moiety, is contacted with a compound of the present invention as defined above to form a reaction mixture.

In some embodiments, the sample may be added to the compound of the present invention, while in other embodiments the aqueous solution comprising the compound of the present invention may be added to the sample.

In some embodiments, the reaction mixture may further comprise an enhancer. The enhancer may comprise CTAB (cetyltrimethylammonium bromide) and other micelle-forming substances. The enhancer may be a natural substance such as bovine serum albumin or similar type biological or protein based molecules or compounds, a fatty-free bovine serum albumin, or any enhancement additive which improves the enhancement of detected chemiluminescence emission effected by the enhancing substance. The enhancer may be a polymer. Representative polymers and their effects as enhancers are set forth in U.S. Patent Nos. 5,145,772 and 5,547,836, which are incorporated herein by reference. These polymers can be used alone, or together with a surfactant additive, to further improve the enhancement value, as disclosed in U.S. Patent No. 5,994,073, also incorporated herein by reference. The enhancer may comprise a polymeric quaternary ammonium salt, polymeric quaternary phosphonium salt or a combination thereof. The polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)] or a combination thereof. The polymeric quaternary phosphonium salt may be poly(vinylbenzyltrimethylphosphonium chloride), poly(vinylbenzyltributylphosphonium chloride), poly(vinylbenzyltrioctylphosphonium chloride), copolymer comprising poly(vinylbenzyltributylphosphonium chloride) and poly(vinylbenzyltrioctylphosphonium chloride), or a combination thereof.

In some embodiments, the enhancer may further comprise an acceptor dye. Amongst these embodiments, the acceptor dye may be a fluorescent dye. In some of these embodiments, the fluorescent dye may be fluorescein, rhodamine, sulforhodamine, ALEXA FLUOR 350, ALEXA FLUOR 405, ALEXA FLUOR 430, ALEXA FLUOR 488, ALEXA FLUOR 532, ALEXA FLUOR 546, or ALEXA FLUOR 555. In some these embodiments, the fluorescent dye may be fluorescein.

In another step of these methods, the reaction mixture is allowed to generate light.

In some embodiments, the light may be observed with the eye or measured using X-ray film or instruments capable of detecting and measuring the light generated. Instruments capable of detecting and measuring the light generated will include, but is not limited to, a luminometer, camera with film or a charge-coupled camera.

In some embodiments, detecting the light generated by the reaction mixture after addition of the compound of the present invention is detected, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light reaches a maximum in less than about 10 minutes. In some of these embodiments, the generation of light reaches a maximum in less than about 5 minutes

In another aspect, the present invention provides kits for detecting the presence or amount of an analyte in a sample comprising a compound of the present invention as defined above and the various embodiments disclosed herein, and a buffer.

In some embodiments, the kits may further comprise an enhancer as described above. In some embodiments, the kits may further comprise an acceptor dye as described above.

In another aspect, the present disclosure provides a method of generating light, comprising the steps of providing the compound having the structure: 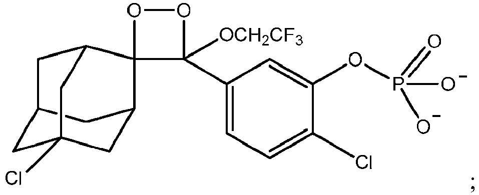 providing an enzyme complex comprising an enzyme moiety which is capable of cleaving the compound; contacting the enzyme complex with the compound to form a reaction mixture; and, allowing the reaction mixture to generate light; provided that when the enzyme complex comprises a solid phase, an antigen is immobilized to the solid phase, and the antigen is protein, then the solid phase is not a membrane or microassay.

In some embodiments, the enzyme moiety may be alkaline phosphatase.

In some embodiments, the enzyme moiety may be an alkaline phosphatase-linked antibody capable of binding an antigen.

In some embodiments, the enzyme moiety may be an alkaline phosphatase-linked antigen.

In some embodiments, the enzyme moiety may be an alkaline phosphatase-linked oligonucleotide.

In some embodiments, the alkaline phosphatase may be non-covalently linked to the antibody, antigen or oligonucleotide. In some of these embodiments, the antibody, antigen or oligonucleotide may be covalently linked to a label and the alkaline phosphatase is covalently linked to a molecule capable of non-covalent binding to the label. In some of these embodiments, the label may be biotin, or a biotin derivative, and the molecule is avidin or strepavidin, while in other of these embodiments, label may be a hapten and the molecule is an antibody capable of binding to the hapten.

In some embodiments, the method may further comprise the steps of providing a sample suspected of comprising an antigen; providing a solid phase comprising a second antibody capable of binding to the antigen; contacting the sample and alkaline phosphatase-linked antibody with the solid phase to form the enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes. In some of these embodiments, the generation of light may reach a maximum in less than about 5 minutes. In some of these embodiments, the method may further comprises the step of removing any unbound alkaline phosphatase-linked antibody from the enzyme complex.

In some embodiments, the method may further comprise the steps of providing a sample suspected of comprising an antigen; providing a solid phase comprising an antibody capable of binding to the antigen; contacting the sample and alkaline phosphatase-linked antigen with the solid phase to form an enzyme complex; and, detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than about 15 minutes. In some of these embodiments, the generation of light may reach a maximum in less than about 10 minutes. In some of these embodiments, the generation of light may reach a maximum in less than about 5 minutes. In some of these embodiments, the method may further comprise the step of removing any unbound alkaline phosphatase-linked antigen from the enzyme complex.

In some embodiments, the method may further comprise the steps of providing a sample suspected of comprising a nucleic acid; immobilizing the nucleic acid to a solid phase, contacting the immobilized nucleic acid and the alkaline phosphatase-linked oligonucleotide to form an enzyme complex; and, detecting the light generated by the reaction mixture after addition of the compound, wherein the generation of light is indicative of the presence of the nucleic acid and the amount of light generated can be correlated to the amount of the nucleic acid present in the sample, and wherein the generation of light at 25°C to 37°C reaches a maximum in less than about 15 minutes. In some of these embodiments, the generation of light may reach a maximum in less than about 10 minutes. In some of these embodiments, the generation of light may reach a maximum in less than about 5 minutes. In some of these embodiments, the method may further comprise the step of removing any unbound enzyme-linked oligonucleotide from the enzyme complex.

In some embodiments, the solid phase may be a bead, test tube, multi-well plate, microarray, gel, membrane, microparticles, nanocrystals, quantum dots and the like. The materials from which these solid phases are made are known in the diagnostic arts.

In some embodiments, the reaction mixture may further comprises an enhancer. The enhancer may comprise CTAB (cetyltrimethylammonium bromide) and other micelle-forming substances. The enhancer may be a natural substance such as bovine serum albumin or similar type biological or protein based molecules or compounds, a fatty-free bovine serum albumin, or any enhancement additive which improves the enhancement of detected chemiluminescence emission effected by the enhancing substance. The enhancer may be a polymer. Representative polymers and their effects as enhancers are set forth in U.S. Patent Nos. 5,145,772 and 5,547,836, which are incorporated herein by reference. These polymers can be used alone, or together with a surfactant additive, to further improve the enhancement value, as disclosed in U.S. Patent No. 5,994,073, also incorporated herein by reference. The enhancer may comprise a polymeric quaternary ammonium salt, polymeric quaternary phosphonium salt or a combination thereof. The polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)] or a combination thereof. The polymeric quaternary phosphonium salt may be poly(vinylbenzyltrimethylphosphonium chloride), poly(vinylbenzyltributylphosphonium chloride), poly(vinylbenzyltrioctylphosphonium chloride), copolymer comprising poly(vinylbenzyltributylphosphonium chloride) and poly(vinylbenzyltrioctylphosphonium chloride), or a combination thereof. In some of these embodiments, the enhancer may comprise a polymeric quaternary ammonium salt, polymeric quaternary phosphonium salt, or a combination thereof. In some of these embodiments, the polymeric quaternary ammonium salt may be poly(vinylbenzyltrimethylammonium chloride), poly[vinylbenzyl(benzyldimethylammonium chloride)], poly[vinyl(benzyltributylammonium chloride)], poly[vinyl(benzyltripentylammonium chloride)], or a combination thereof. In some of these embodiments, the polymeric quaternary phosphonium salt may be poly(vinylbenzyltrimethylphosphonium chloride), poly(vinylbenzyltributylphosphonium chloride), poly(vinylbenzyltrioctylphosphonium chloride), a copolymer comprising poly(vinylbenzyltributylphosphonium chloride) and poly(vinylbenzyltrioctylphosphonium chloride), or a combination thereof.

In some embodiments, the enhancer may further comprise an acceptor dye. In some of these embodiments, the acceptor dye may be fluorescein, rhodamine, sulforhodamine, ALEXA FLUOR 350, ALEXA FLUOR 405, ALEXA FLUOR 430, ALEXA FLUOR 488, ALEXA FLUOR 532, ALEXA FLUOR 546, or ALEXA FLUOR 555.

The following examples are intended to illustrate the invention.

### Instruments.

96/384-well HydroSpeedplate washer (TecanGroup Ltd., Switzerland), Orion-II 96/384-well microplate luminometer (Titertek Instrument Berthold Technologies, Huntsville, AL) were used for measuring chemiluminescence.

### Beads and Microplates.

Dynabeads® (Life Tech Dynal AS, Norway), Corning 96-well polystyrene microplate, flat bottom, high binding, solid white (Corning, 3922), and Corning 96-well microplate, round bottom, solid white (Corning, 3355) were obtained.

### Chemiluminescent Substrates and Enhancers.

CSPD® and CDP-Star® were obtained Life Technologies, San Diego, California.

AP-5 chemiluminescent substrate (for alkaline phosphatase) was obtained Lumigen, Inc., Southfield, Michigan. This is referred to herein as Competitor's Substrate D or Competitor's AP Substrate D, Competitor's Flash Substrate D, or Vendor X's Substrate D

LumiGLO Reserve™ Chemiluminescent Substrate (for HRP) was obtained from KPL, Inc. (Gaithersburg, Maryland). This is referred to herein as Competitor's HRP Substrate or Competitor's Glow Substrate E.

Sapphire-II™ Luminescence Enhancer (Sapphire-II), Sapphire-III™ Luminescence Enhancer (Sapphire-III), Emerald™-II Luminescence Enhancer (Emerald-II), and Emerald™-III Luminescence Enhancer (Emerald-III) were obtained from Applied Biosystems Inc., Foster City, California.

### Purified alkaline phosphatase assay.

100 µL of substrate formulation was added to 10 µL of serial dilutions of alkaline phosphatase (BBI BioZyme, ALPI12G) and white microtiter plate was read kinetically 2 minutes after substrate addition.

### Biotinylated-alkaline phosphatase coupled in streptavidin-coated M-280 Tosylactivated Dynabeads®.

10 µL of washed beads were incubated with 40 µL of 10-fold serial dilutions of biotinylated alkaline phosphatase, (BioLabs, N7022S), for 40 min, at 25°C. Beads were separated with a donut magnet (Life Tech Ambion's magnet cat #: AM10050) for 2 to 3 min and washed 3x with 1x PBS containing 1% BSA.

100 µL of substrate formulation was added and plate was read kinetically 2 minutes after substrate addition

### Sandwich ELISA using microplate as solid support for capturing antibodies.

A monoclonal antibody to human IL-6 (R&D Systems, MAB206) was immobilized to a 96-well microplate to bind to standard human recombinant IL-6 (R&D Systems, 206-IL). A biotinylated anti-human IL-6 (R&D Systems, BAF206) was added which binds to the human recombinant IL-6 bound on the plate, and then a streptavidin alkaline phosphatase(Jackson ImmunoResearch,16-050-084) was added. Independent of chemistry detection, the immunoassay procedure was identical. Substrate formulation was added to the washed wells and plate read kinetically 37 seconds after substrate addition.

### Troponin sandwich ELISA using Dynabeads® as solid support for capturing antibodies.

50 µL of human cardiac troponin I (HyTest Ltd., 8T53) in human serum (Sigma, S7023), was incubated for 10 minutes, at 25 ° C with 50 µL of mouse α-human cardiac Troponin I (α-cTnI), SDIX (clone TPC6) antibody conjugated to alkaline phosphatase using LYNX Rapid Alkaline Phosphatase Antibody Conjugation Kit (AbDSerotec, LNK012AP).

100 µL of Dynabeads® coupled with ®-human cardiac Troponin I (α-cTnI) monoclonal antibody, (HyTest Ltd., 4T21, Mab560), was incubated with the above sample for 30 minutes, at 37°C, with shaking. Beads were separated with a donut magnet (Life Tech Ambion's magnet cat #: AM10050) for 2 to 3 minutes and washed 3x with TBS-Tween wash buffer.

100 µL of substrate formulation was added and plate read kinetically 2 minutes after substrate addition.

### Example 1

### Synthesis of Substrate A

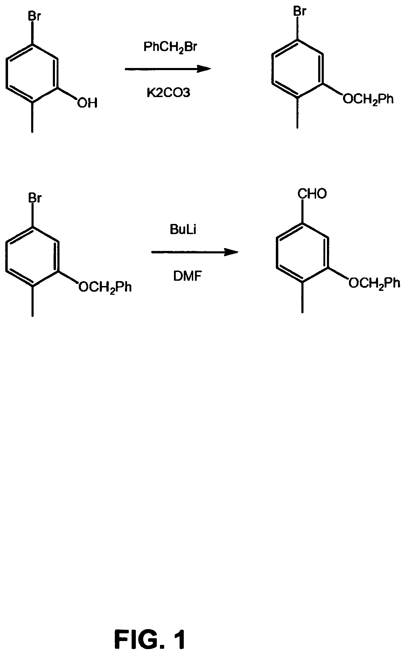

Substrate A was made by the reaction scheme outlined in FIG. 1 through FIG. 4.

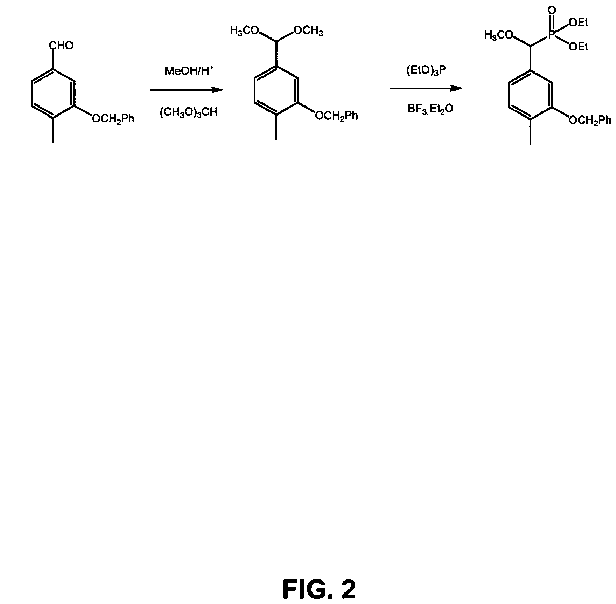

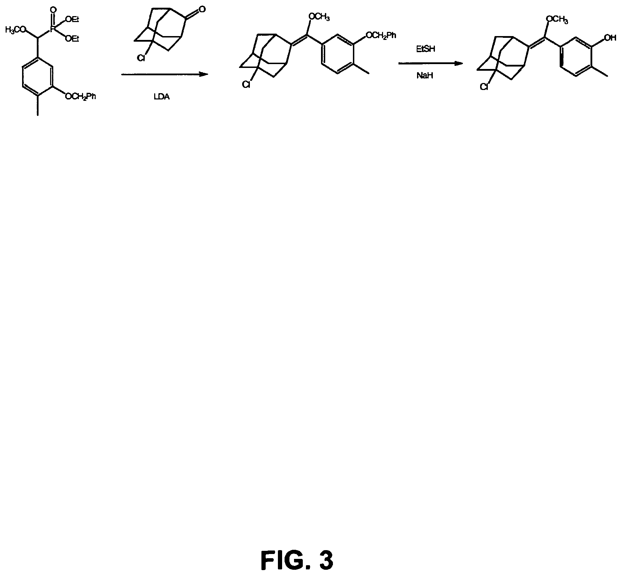

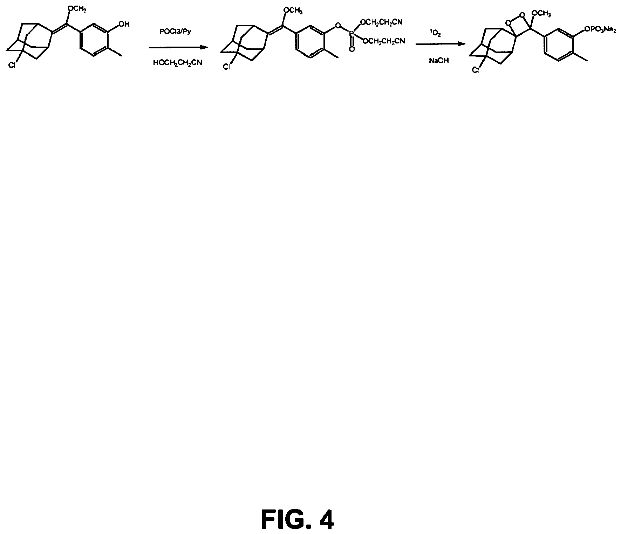

4-Me-3-OBN-benzaldehyde was made as shown in FIG. 1. 17.56 g of starting material yielded 13.6 g of the final product. 4-Me-Phe phosphonate was made starting with 4-Me-3-OBN-benzaldehyde and as shown in FIG. 2. 13.6 g of starting material yielded 17.7 g of the final product. The enol ether precursor of Substrate A was made starting with 4-Me-Phe phosphonate by the reaction scheme shown in FIG. 3. 5.6 g of starting material yielded 2.1 g of the final product. Substrate A was made starting with the enol ether precursor of Substrate A as shown in FIG. 4. 1.5 g of starting material yielded 1.37 g of the final product.

The compounds of the present invention can be made by the reaction scheme described above with modifications known to those skilled artesian.

### Example 2

### Kinetic Study of Dioxetane Substrates done in microtiter plates at 25°C

The kinetics of light emission by CSDP, CDP-Star, Substrate A and Substrate B were evaluated using the "Purified alkaline phosphatase assay" protocol run at 25° C.

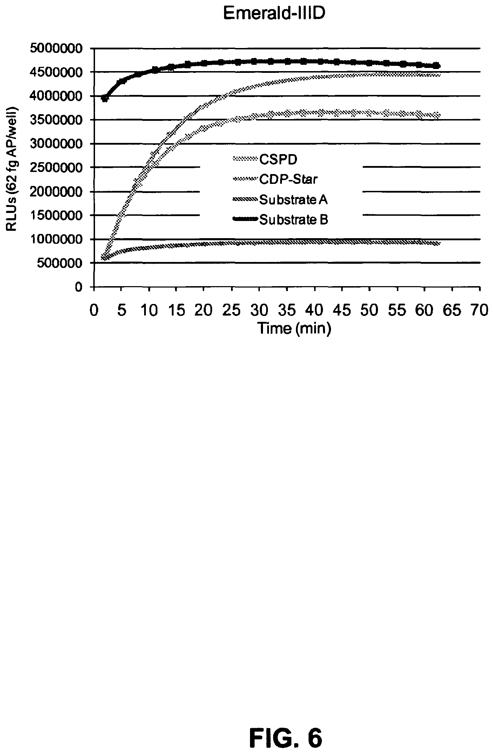

FIG. 6 shows the results of the observed time course of light emission. The results show that Substrates A and B reached maximum light emission in 5 to 10 minutes, while CDP-Star did so after 30 to 60 minutes. All substrates tested showed signal stability of hours after reaching maximum light emission. (See Table to FIG. 6)

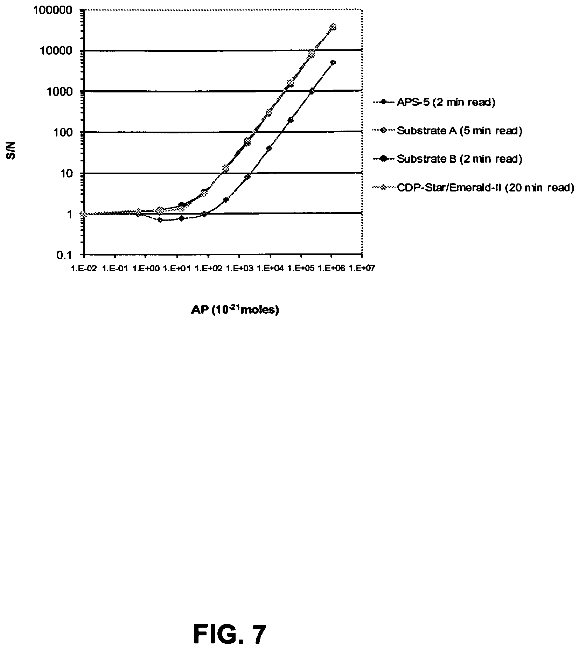

FIG. 7 shows the sensitivity curves run using varying concentrations of alkaline phosphatase with CDP-Star, Substrate A, Substrate B and AP-5, reading the total emission at the specified time. Sensitivity was defined as lowest concentration of alkaline phosphatase at which the signal-to-noise ratio was equal to 2. The sensitivity of CDP-Star, Substrate A, and Substrate B was found to be greater than 10-times that of AP-5. (See Table to FIG. 7)

### Example 3

### Kinetic Evaluation of Substrate A, CSPD, and CDP-Star in IL-6 Immunoassay done in microtiter plates at 25°C

The kinetics of light emission by Substrate A, CSDP, and CDP-Star was evaluated in the "Sandwich ELISA using microplate as solid support for capturing antibodies" protocol at 25°C.

**Table to FIG. 6**

| **Time required to reach maximum light emission (peak) at 25°C:** | |
|---|---|
| **CDP-Star/Emerald-II** | **30-60 min** |
| **Substrate A** | **5-10 min** |
| **Substrate B** | **5-10 min** |

| **Signal stability after reaching maximum light emission at 25° C:** | |
|---|---|
| **All substrates** | **hours** |

**Table to FIG. 7**

| **CL Detection** | **Lumigen**'**s APS-5 (Substrate D)** | **Substrate A** | **Substrate B** | **CDP-Star/Emerald-II** |
|---|---|---|---|---|
| Amount of AP at S/N =2 | 0.36 attomole | 0.03 attomole | 0.03 attomole | 0.03 attomole |

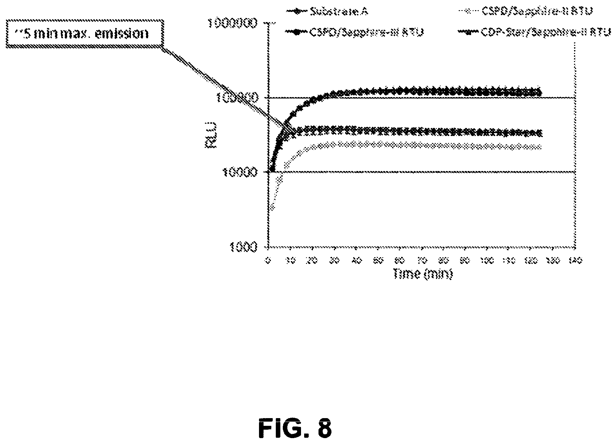

FIG. 8 shows the time course of light emission with Substrate A when compared to CSPD with Sapphire-II, CSPD with Sapphire-III, and CDP-Star with Sapphire-II. The maximum light emission with Substrate A is at about 5 minutes versus 30-60 minutes with CSPD and CDP-Star.

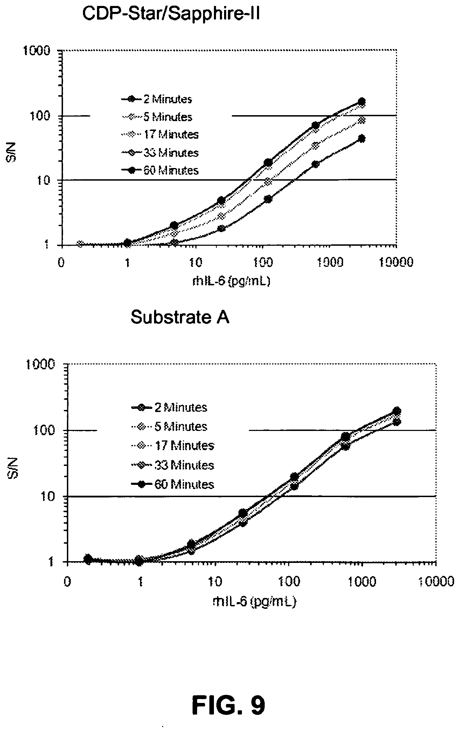

FIG. 9 shows the signal to noise ratios at 25 ° C for CDP-Star with Sapphire-II and Substrate A. With CDP-Star maximum performance requires waiting 15 minutes after addition of this substrate. With Substrate A, the early glow signal provides stable signal-to-noise ratios over the observed time course of the assay rendering its ideal for microplate ELISAs.

### Example 4

### Kinetic Evaluation of Substrates A, B, C in IL-6 Immunoassay done in microtiter plates at 37°C

The kinetics of light emission by Substrates A, B, C was evaluated in the "Sandwich ELISA using microplate as solid support for capturing antibodies" protocol at 37°C.

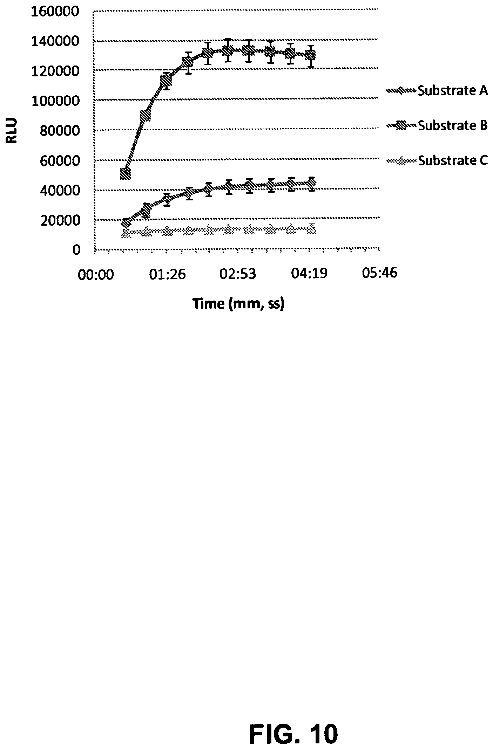

FIG. 10 shows the kinetics of 24 pg/mL of IL-6 in a immunoassay which is representative of a relevant low analyte concentration. The time to reach maximum light emission for
Substrates A, B and C are 3 minutes, 2 minutes and immediate, respectively. (See Table to FIG. 10)

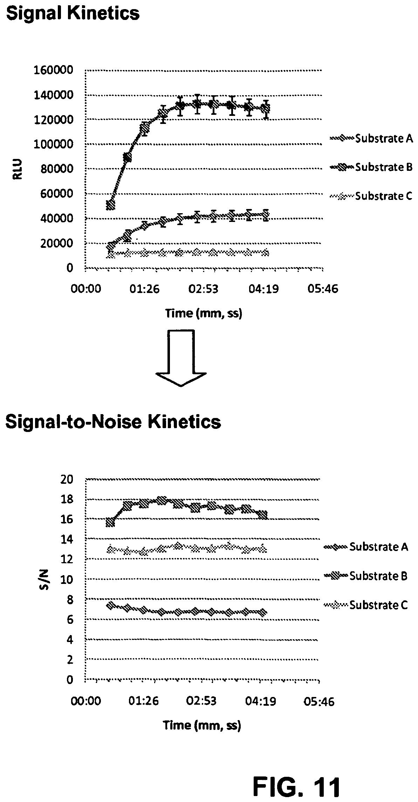

FIG. 11 shows the kinetics for the the signal (light emission) and signal-to-noise for Substrates A, B and C. The constant signal-to-noise ratios observed for Substrates A, B and C enables flexibility in time of detection which renders immunoassay using these substrates compatible with most instrument formats.

### Example 5

### Sensitivity Comparison of Substrates A, B, and C in an Immunoassay System done on microtiter plates at 37°C

The sensitivity using Substrates A, Substrate B, Substrate C, CDP-Star with Emerald-II, Competitor's AP Substrate D, and Competitor's HRP Substrate was

**Table to FIG. 10**

| **Time required to reach maximum light emission (peak) at 37°C:** | |
|---|---|
| **Substrate A** | **3 min** |
| **Substrate B** | **2 min** |
| **Substrate C** | **Immediate** |

evaluated in the "Sandwich ELISA using microplate as solid support for capturing antibodies" protocol at 37 ° C.

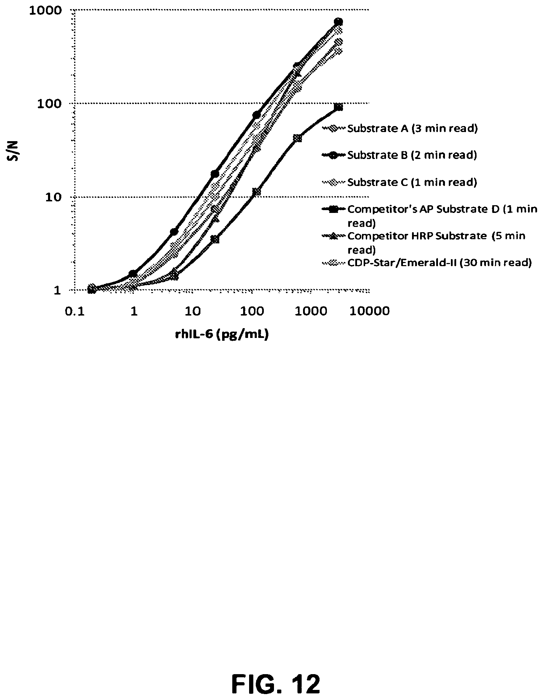

FIG. 12 shows that Substrates A, B and C provide a sensitivity 5 to 8 times greater than that observed with Competitor's AP Substrate D and Competitor's HRP Substrate. Sensitivity is defined the lowest amount ofhIL-6 detect at a signal-to-noise of 2. (See Table to FIG. 12)

### Example 6

### Shelf Life Stability of Substrate A

The maximum light emission by Substrate A samples stored at -20 °C, 4 °C, and 37 °C were evaluated using the "Purified alkaline phosphatase assay" protocol. Samples stored at these temperatures were assayed on the same day over the course of 80 days.

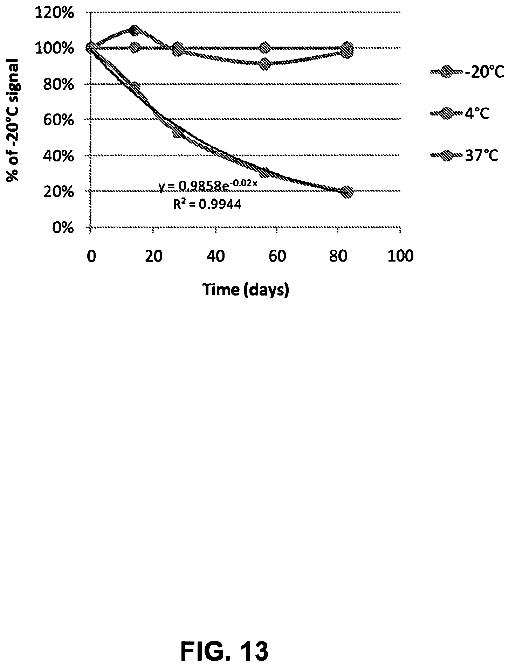

FIG. 13 shows the result of this study. Based on the extrapolation of the 37 ° C accelerated curve, the real time (4 °C) stability of Substrate A is estimated to 14 months.

### Criteria:

1. The data in this graph has been normalized versus the -20°C signal intensity at each time point.
2. Stability is defined as the time it takes for the signal intensity to decay to 80% of -20°C control.

### Example 7

### Kinetics Evaluation of Substrates A, B and C on Dynabeads M-280 beads at room temperature

The kinetics of light emission and signal-to-noise of Substrate A, Substrate B, Substrate C, CDP-Star with Emerald-II, Competitor's Flash Substrate D, and Competitor's Flow Substrate E was evaluated using Strepavidin-labeled Dynabeads M-280 Tosylactivated beads bound with biotin-labeled alkaline phosphatase. 20 µg of the Strepavidin-labeled Dynabeads was bound with 0.04 ng of biotin-labeled alkaline phosphatase. The evaluation was done at room temperature.

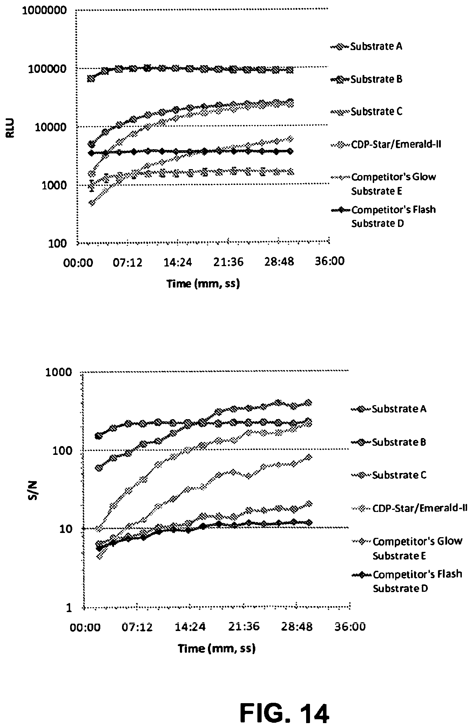

FIG. 14 shows the results of this evaluation. Substrate B stood out in having a fast ramp up to maximum light emission and sustained a constant signal-to-noise over the time course of observation. Substrate A ramped up to maximum light emission more slowly than that for Substrate B, while Substrate C ramped up to maximum light emission very quickly.

**Table to FIG. 12**

| **Rank** | **1** | **2** | **4** | **3** | **5** | **6** |
|---|---|---|---|---|---|---|
| **Detection System** | **Substrate B** | **Substrate A** | **CDP-Star/ Emerald-II** | **Substrate C** | **Competitor's HRP Substrate** | **Competitor's APS-5 (AP Substrate)** |
| [IL-6] at S/N = 2 | 1.6 pg/mL | 2.6 pg/mL | 3 pg/mL | 3.5 pg/mL | 8 pg/mL | 12 pg/mL |

### Example 8

### Sensitivity curves for Substrates A, B and C on Dynabeads M-280 beads at room temperature

The sensitivity of Substrates A, Substrate B, Substrate C, CDP-Star with Emerald-II, Competitor's Flash Substrate D, and Competitor's Glow Substrate E was evaluated using the "Biotinylated-alkaline phosphatase coupled in streptavidin-coated M-280 Tosylactivated Dynabeads®" protocol using Strepavidin-labeled Dynabeads M-280 Tosylactivated beads bound with biotin-labeled alkaline phosphatase. 20 µg of the Strepavidin-labeled Dynabeads was bound with variable amounts of biotin-labeled alkaline phosphatase. The evaluation was done at the optimum read time for the respective substrates and at room temperature.

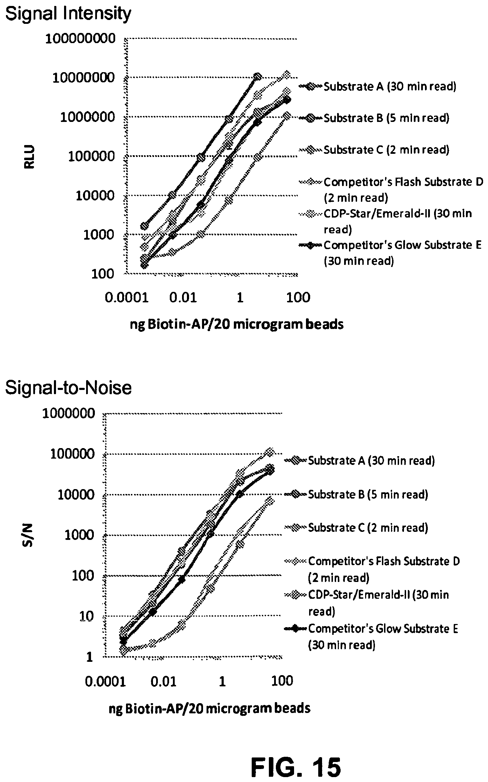

FIG. 15 shows the results of this evaluation. Substrate A provides a high signal and signal-to-noise rations. Substrate B provides the highest signal and high signal-to-noise ratios. Substrate C sensitivity was similar to the Competitor's Flash Substrate D. Substrate B performs similar or better than the dioxetanes evaluated.

The sensitivity of Substrates A, Substrate B, Substrate C, CDP-Star with Emerald-II, Competitor's Flash Substrate D, and Competitor's Glow Substrate E was evaluated as above, where the signal was read at 2 minutes.

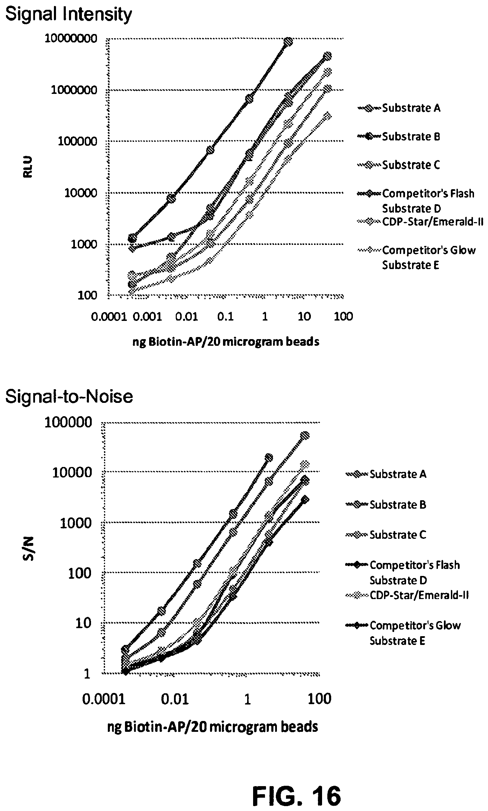

FIG. 16 shows the results of this evaluated. Substrate B performs better or similarly to the other substrates at their best optimum read times.

### Example 9

### Light Emission Kinetics and Signal-to-Noise of Substrates A, Substrate B and CDP-Star on Dynabeads MyOne Carboxylic Acid beads at 37 o C

The kinetics of light emission and signal-to-noise of Substrate A with Emerald-III, Substrate B, and CDP-Star with Emerald-II was evaluated using the "Troponin sandwich ELISA using Dynabeads® as solid support for capturing antibodies" protocol, using Dynabeads MyOne Carboxylic Acid beads. The protocol was run using 16 pg/mL cTn1 and at 37°C.

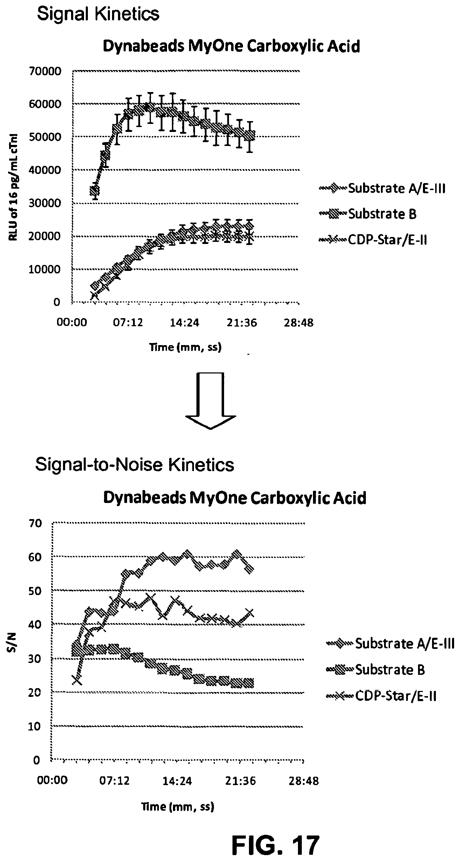

FIG. 17 shows the results of this evaluation. The signal-to noise ratio were variable over time depending upon the substrate formulation. Substrate B signal-to-noise ratios were constant during the first 10 minutes.

### Example 10

### Immunoassay Performance at Optimum Read Time and at 37°C

The performance of Substrate A with Emerald-III, Substrate B, and CDP-Star with Emerald-II was evaluated using the "Troponin sandwich ELISA using Dynabeads® as solid support for capturing antibodies" protocol at 37°C.

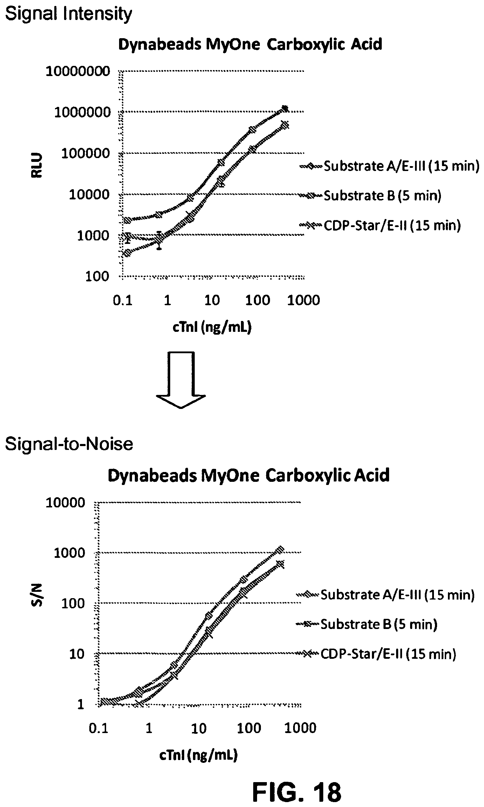

FIG. 18 shows the results of this evaluation. Substrate B provided the highest signal and performs similarly to the other substrate formulations test in signal-to-noise ratio due to high background.

### Example 11

### Evaluation of Beads and Luminescence Enhancers in an Immunoassay using Substrate A

DynaBeads M-280 Tosylactivated, Dynabeads MyOne Carboxylic Acid, and Dynabeads M-270 Epoxy were coupled with α-human cardiac Troponin I (α-cTnI) monoclonal antibody, (HyTest Ltd., 4T21, Mab560).

Substrate A with Sapphire-III and Substrate with Emerald-III were evaluated for assay performance using the "Troponin sandwich ELISA using Dynabeads® as solid support for capturing antibodies" protocol at 37°C.

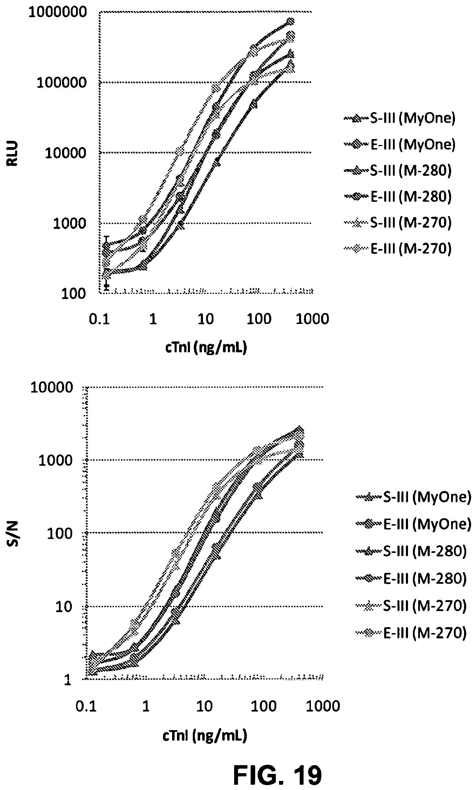

FIG. 19 shows the results of this evaluation. DynaBeads M-270 Epoxy beads provided the highest assay performance. Emerald-III Luminescence Enhancer provided the higher signal and somewhat better sensitivity with all bead types tested. S-III refers to Sapphire-III, while E-III refers to Emerald-III.

### Example 12

### Troponin Sandwich Beads Immunoassay

Dynabeads M-270 Epoxy was coupled with α-human cardiac Troponin I (α-cTnI) monoclonal antibody, (HyTest Ltd., 4T21, Mab560). The performance in an immunoassay of Substrate A, Substrate B, Substrate C, CDP-Star with Emerald-II, and Vendor X's Substrate D was evaluated using the "Troponin sandwich ELISA using Dynabeads® as solid support for capturing antibodies" protocol at 37°C.

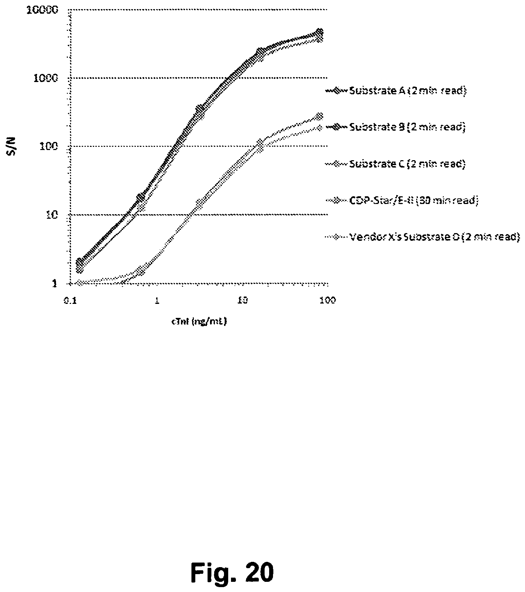

FIG. 20 shows the results of the evaluation. At a signal-to-noise ratio of 2, the Substrates A, B or C, provided a low-end detection of as low as 0.1 ng/well of human cardiac troponin I (cTn1) in human serum, and a dynamic range of about 3 logs. Vendor X's Substrate D provided a low-end detection of 0.6 ng/well of cTn1 and a dynamic range of about 3 logs.

## Claims

1. A compound of having one of the following structures 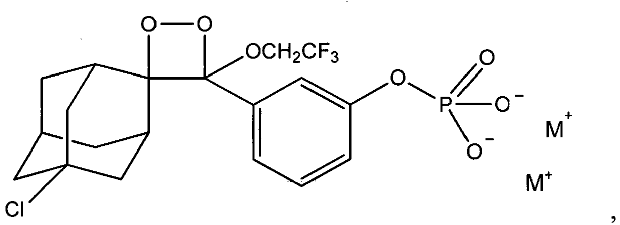 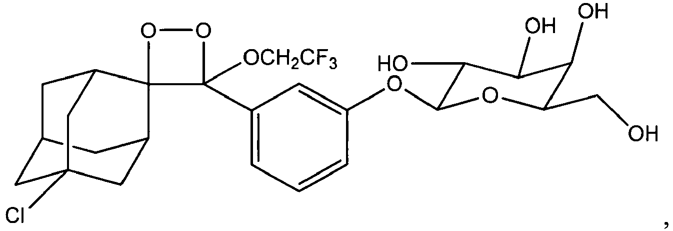 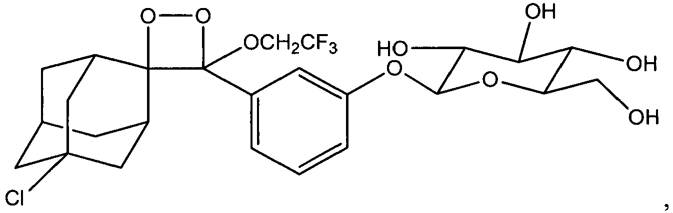 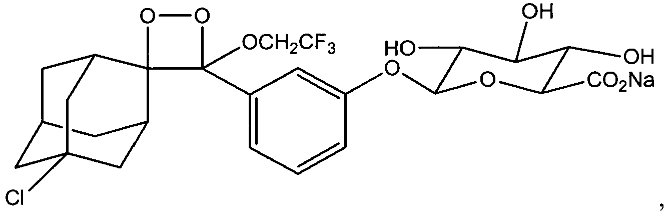 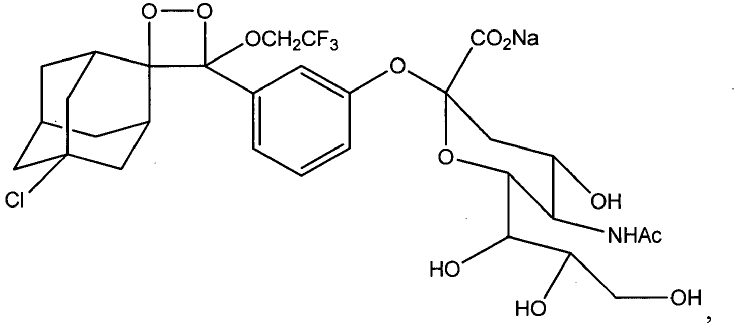 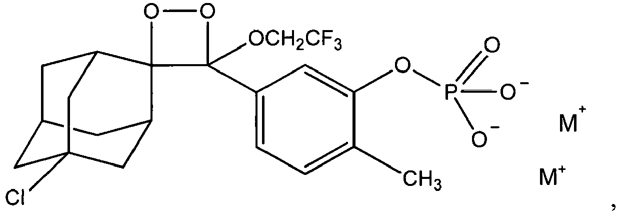 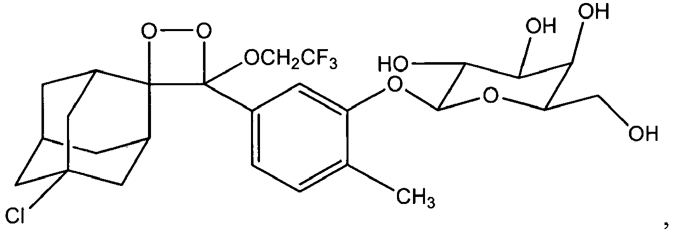 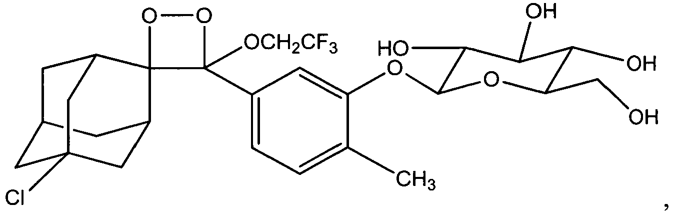 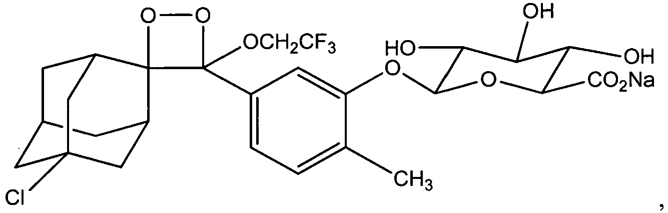 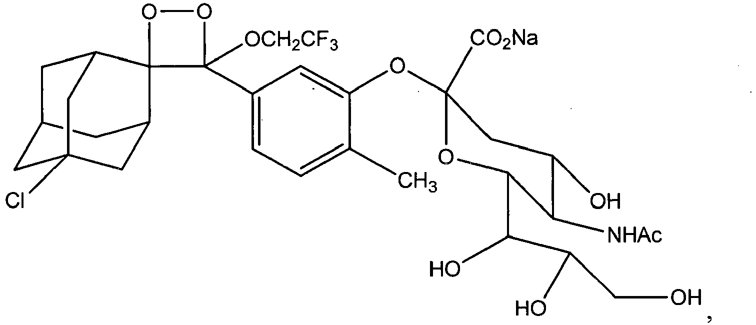 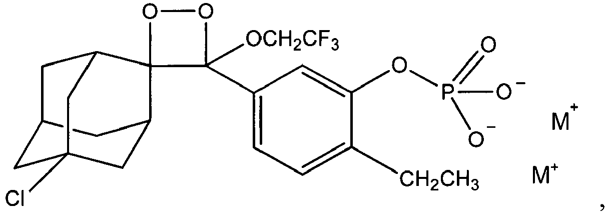 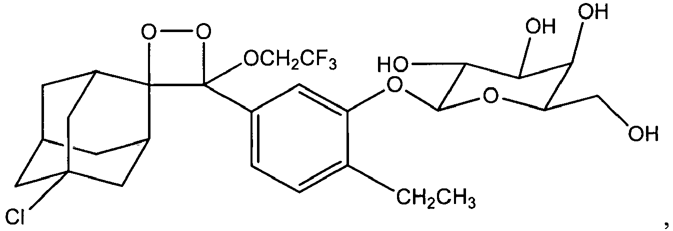 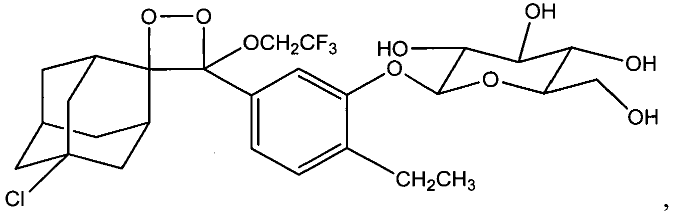 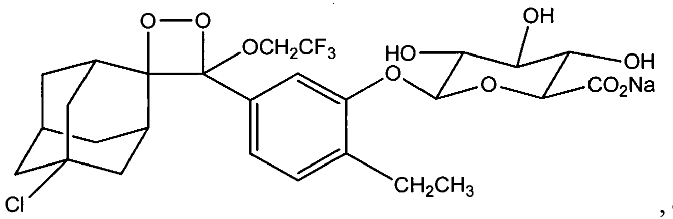 or 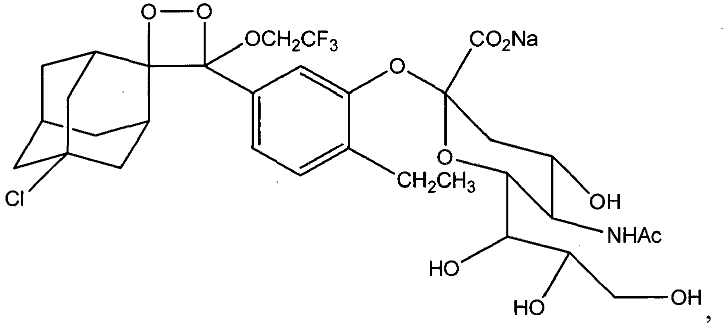 wherein M⁺ represents a cation of an alkali metal, e.g., sodium or potassium, ammonium, or a C₁₋₇ alkyl, aralkyl or aromatic quaternary ammonium cation, N(D₃)₄⁺ in which each D₃ can be alkyl, e.g., methyl or ethyl, aralkyl, e.g., benzyl, or form part of a heterocyclic ring system, e.g., pyridinium, or particularly the disodium salt.

2. A method for generating light, comprising the steps of:
(a) providing the compound of claim 1;
(b) providing an enzyme complex comprising an enzyme moiety which is capable of cleaving the compound;
(c) contacting the enzyme complex with the compound to form a reaction mixture; and,
(d) allowing the reaction mixture to generate light.

3. The method of claim 2, wherein the enzyme moiety comprises a hydrolytic enzyme.

4. The method of claim 3, wherein the hydrolyic enzyme is alkaline phosphatase, β-galactosidase, β-glucosidase, β-glucuronidase, or neuraminidase.

5. The method of claim 4, further comprising the step of detecting the light generated by the reaction mixture after addition of the compound, wherein the generation of light is indicative of the presence of the enzyme and the amount of light generated can be correlated to the amount of the enzyme present in the sample, and wherein the generation of light at 25 ° C to 37 ° C reaches a maximum in less than 15 minutes.

6. The method of claim 2, wherein the reaction mixture further comprises an enhancer comprising a polymeric quaternary ammonium salt, polymeric quaternary phosphonium salt, or a combination thereof.

7. The method of claim 6, wherein the enhancer further comprises an acceptor dye.

8. An assay method for determining the presence and/or amount of an enzyme in a sample, comprising the steps of:
(a) providing the compound of claim 1;
(b) providing a sample suspected of comprising the enzyme which is capable of cleaving the compound so that it decomposes and generates light;
(c) contacting the sample with the compound to form a reaction mixture; and,
(d) detecting the light generated by the reaction mixture after addition of the compound, wherein the generation of light is indicative of the presence of the enzyme and the amount of light generated can be correlated to the amount of the enzyme present in the sample, and wherein the generation of light at 25 ° C to 37 ° C reaches a maximum in less than 15 minutes.

9. An assay method for determining the presence and/or amount of an antigen in a sample, comprising the steps of:
(a) providing the compound of claim 1;
(b) providing a sample suspected of comprising the antigen;
(c) providing an enzyme-linked antigen comprising the antigen and an enzyme capable of cleaving the compound so that it decomposes and generates light;
(d) providing a solid phase comprising an antibody capable of binding to the antigen;
(e) contacting the sample and enzyme-linked antigen with the solid phase to form an enzyme complex;
(f) contacting the enzyme complex with the compound to form a reaction mixture; and,
(g) detecting the light generated by the reaction mixture after addition of the compound, wherein the generation of light is indicative of the presence of the antigen and the amount of light generated can be correlated to the amount of the antigen present in the sample, and wherein the generation of light at 25°C to 37°C reaches a maximum in less than 15 minutes.

10. An assay method for determining the presence and/or amount of a nucleic acid in a sample, comprising the steps of:
(a) providing the compound of claim 1;
(b) providing a sample suspected of comprising the nucleic acid;
(c) immobilizing the nucleic acid to a solid phase;
(d) providing an enzyme-linked oligonucleotide comprising an oligonucleotide capable of hydridizing to the nucleic acid and an enzyme capable of cleaving the compound so that it decomposes and generates light;
(e) contacting the immobilized nucleic acid and enzyme-linked oligonucleotide to form an enzyme complex;
(f) contacting the enzyme complex with the compound to form a reaction mixture; and,
(g) detecting the light generated by the reaction mixture after addition of the compound, wherein the generation of light is indicative of the presence of the nucleic acid and the amount of light generated can be correlated to the amount of the nucleic acid present in the sample, and wherein the generation of light at 25°C to 37°C reaches a maximum in less than 15 minutes.

11. A kit for detecting the presence and/or amount of an analyte in a sample comprising:
(a) the compound of claim 1, and
(b) a buffer.

## Patentansprüche

1. Verbindung mit einer der folgenden Strukturen 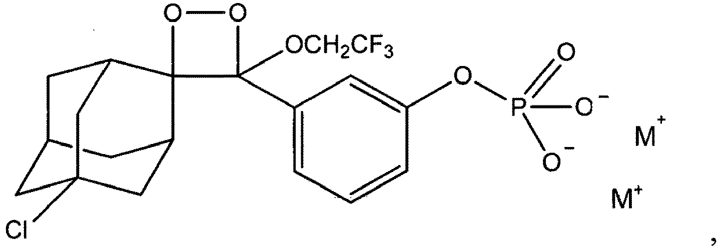 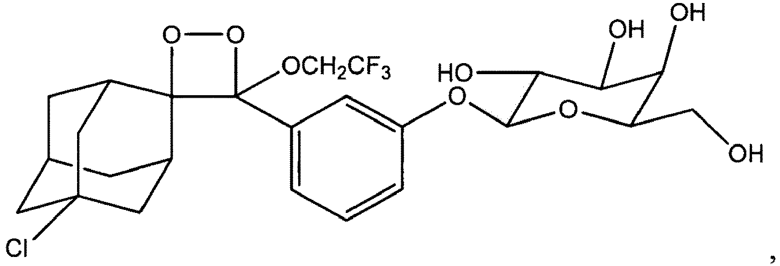 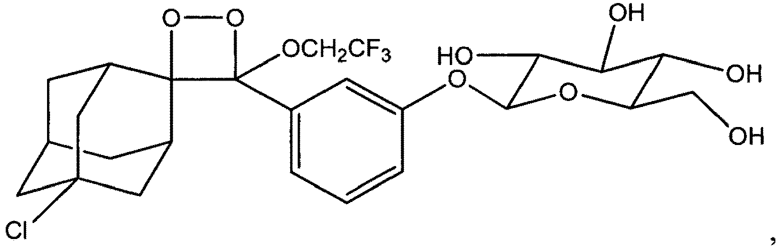 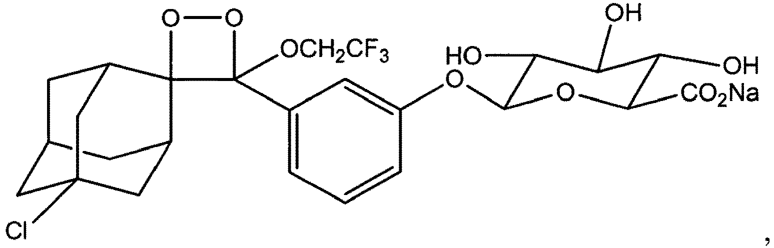 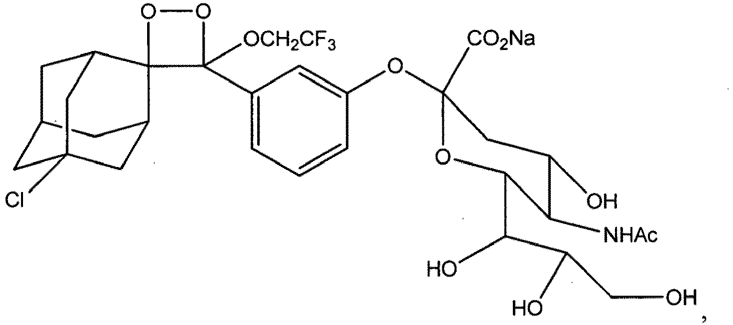 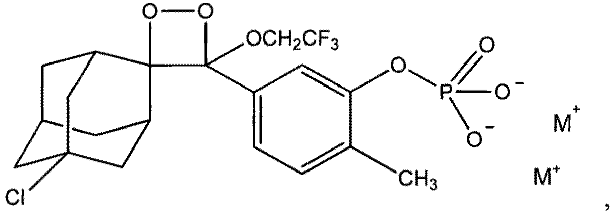 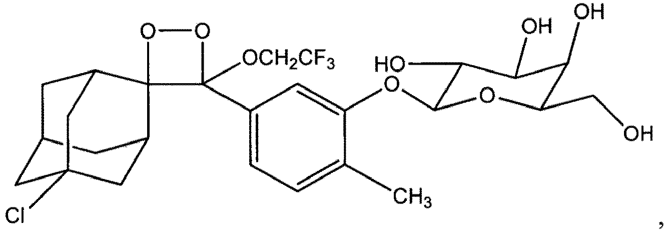 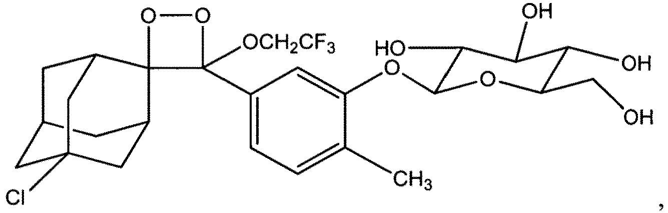 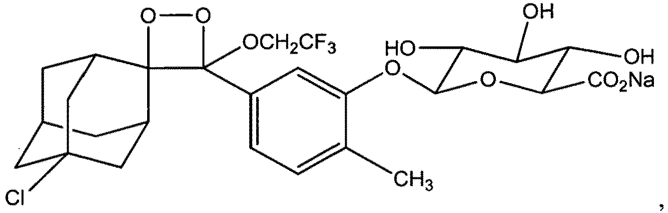 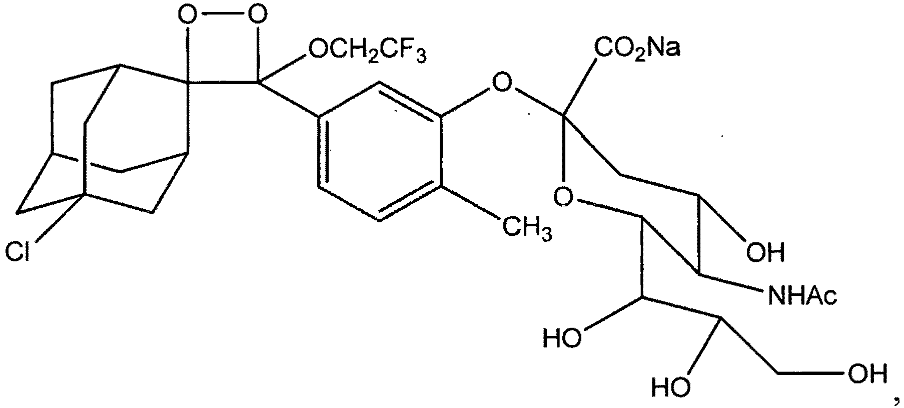 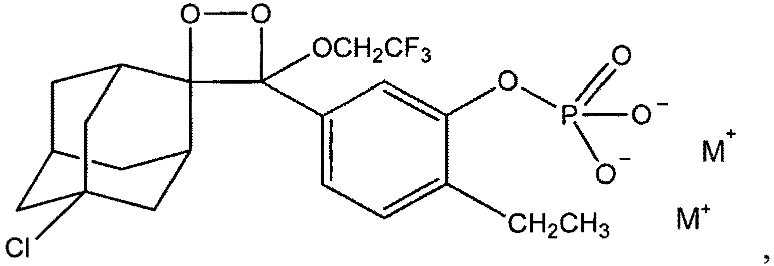 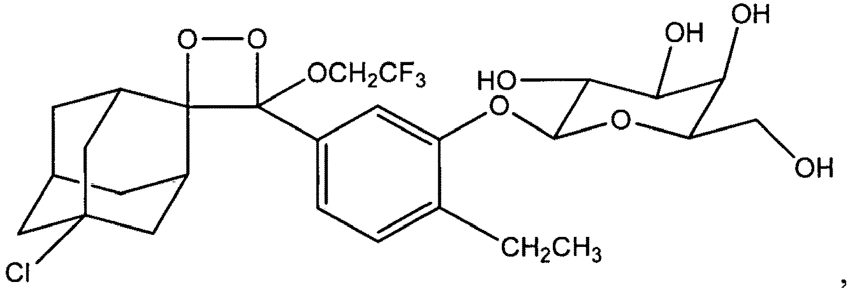 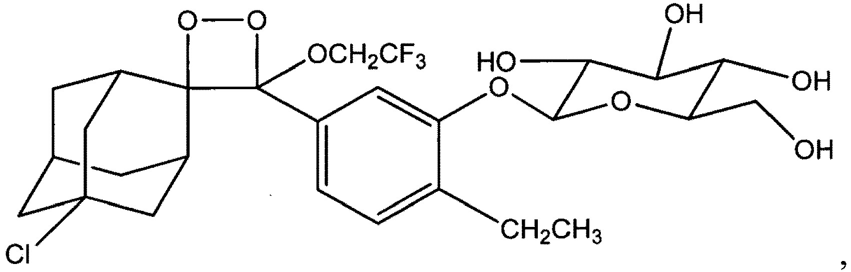 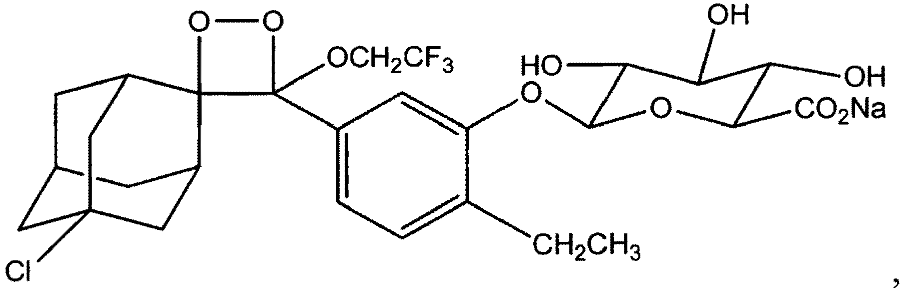 oder 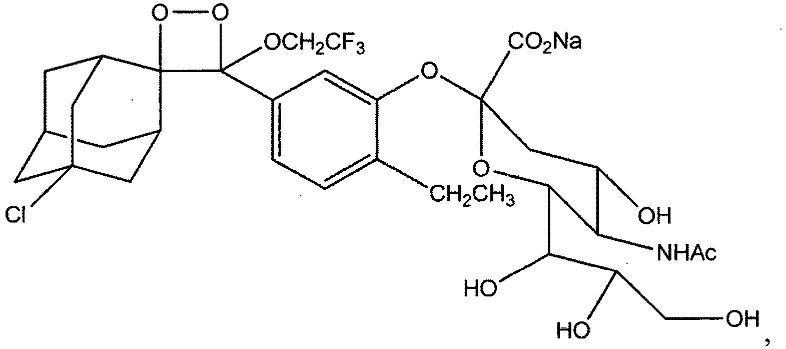 worin M⁺ ein Kation eines Alkalimetalls, z. B. Natrium oder Kalium, Ammonium oder ein C₁₋₇-Alkyl, ein Aralkyl oder ein aromatisches quaternäres Ammoniumkation, N(D₃)₄⁺ darstellt, in dem jedes D₃ ein Alkyl, z. B. Methyl oder Ethyl, ein Aralkyl, z. B. Benzyl, sein oder Teil eines heterozyklischen Ringsystems, z. B. Pyridinium, oder insbesondere das Dinatriumsalz bilden kann.

2. Verfahren zum Erzeugen von Licht, das die folgenden Schritte umfasst:
(a) Bereitstellen der Verbindung von Anspruch 1;
(b) Bereitstellen eines Enzymkomplexes, der einen Enzymanteil umfasst, der in der Lage ist, die Verbindung zu spalten;
(c) Inkontaktbringen des Enzymkomplexes mit der Verbindung, um ein Reaktionsgemisch zu bilden; und
(d) Zulassen, dass das Reaktionsgemisch Licht erzeugt.

3. Verfahren nach Anspruch 2, wobei der Enzymanteil ein hydrolytisches Enzym umfasst.

4. Verfahren nach Anspruch 3, wobei das hydrolytische Enzym alkalische Phosphatase, β-Galactosidase, β-Glucosidase, β-Glucoronidase oder Neuraminidase ist.

5. Verfahren nach Anspruch 4, das ferner den Schritt des Erfassens des von dem Reaktionsgemisch nach Zugabe der Verbindung erzeugten Lichts umfasst, wobei die Erzeugung von Licht auf das Vorhandensein des Enzyms hindeutet und die Menge an erzeugtem Licht mit der Menge des in der Probe vorhandenen Enzyms korreliert werden kann, und wobei die Erzeugung von Licht bei 25°C bis 37°C ein Maximum in weniger als 15 Minuten erreicht.

6. Verfahren nach Anspruch 2, wobei das Reaktionsgemisch ferner einen Enhancer umfasst, der ein polymeres, quaternäres Ammoniumsalz, ein polymeres, quaternäres Phosphoniumsalz oder eine Kombination davon umfasst.

7. Verfahren nach Anspruch 6, wobei der Enhancer einen Akzeptorfarbstoff umfasst.

8. Assayverfahren zum Bestimmen des Vorhandenseins und/oder der Menge eines Enzyms in einer Probe, das die folgenden Schritte umfasst:
(a) Bereitstellen der Verbindung von Anspruch 1;
(b) Bereitstellen einer Probe, von der man vermutet, dass sie das Enzym umfasst, das in der Lage ist, die Verbindung zu spalten, sodass sie zerfällt und Licht erzeugt;
(c) Inkontaktbringen der Probe mit der Verbindung, um ein Reaktionsgemisch zu bilden; und
(d) Erfassen des von dem Reaktionsgemisch nach Zugabe der Verbindung erzeugten Lichts, wobei die Erzeugung von Licht auf das Vorhandensein des Enzyms hindeutet und die Menge an erzeugtem Licht mit der Menge des in der Probe vorhandenen Enzyms korreliert werden kann, und wobei die Erzeugung von Licht bei 25°C bis 37°C ein Maximum in weniger als 15 Minuten erreicht.

9. Assayverfahren zum Bestimmen des Vorhandenseins und/oder der Menge eines Antigens in einer Probe, das die folgenden Schritte umfasst:
(a) Bereitstellen der Verbindung von Anspruch 1;
(b) Bereitstellen einer Probe, von der man vermutet, dass sie das Antigen umfasst;
(c) Bereitstellen eines emzymgebundenen Antigens, das das Antigen und ein Enzym umfasst, das in der Lage ist, die Verbindung zu spalten, sodass sie zerfällt und Licht erzeugt;
(d) Bereitstellen einer Festphase, die einen Antikörper umfasst, der in der Lage ist, an das Antigen zu binden;
(e) Inkontaktbringen der Probe und des enzymgebundenen Antigens mit der Festphase, um einen Enzymkomplex zu bilden;
(f) Inkontaktbringen des Enzymkomplexes mit der Verbindung, um ein Reaktionsgemisch zu bilden; und
(g) Erfassen des von dem Reaktionsgemisch nach Zugabe der Verbindung erzeugten Lichts, wobei die Erzeugung von Licht auf das Vorhandensein des Antigens hindeutet und die Menge an erzeugtem Licht mit der Menge des in der Probe vorhandenen Antigens korreliert werden kann, und wobei die Erzeugung von Licht bei 25°C bis 37°C ein Maximum in weniger als 15 Minuten erreicht.

10. Assayverfahren zum Bestimmen des Vorhandenseins und/oder der Menge einer Nukleinsäure in einer Probe, das die folgenden Schritte umfasst:
(a) Bereitstellen der Verbindung von Anspruch 1;
(b) Bereitstellen einer Probe, von der man vermutet, dass sie die Nukleinsäure umfasst;
(c) Immobilisieren der Nukleinsäure an eine Festphase;
(d) Bereitstellen eines enzymgebundenen Oligonukleotids, das ein Oligonukleotid umfasst,das in der Lage ist, an die Nukleinsäure zu hybridisieren, und ein Enzym, das in der Lage ist, die Verbindung zu spalten, sodass sie zerfällt und Licht erzeugt;
(e) Inkontaktbringen der immobilisierten Nukleinsäure und des enzymgebundenen Oligonukleotids, um einen Enzymkomplex zu bilden;
(f) Inkontaktbringen des Enzymkomplexes mit der Verbindung, um ein Reaktionsgemisch zu bilden; und
(g) Erfassen des von dem Reaktionsgemisch nach Zugabe der Verbindung erzeugten Lichts, wobei die Erzeugung von Licht auf das Vorhandensein der Nukleinsäure hindeutet und die Menge an erzeugtem Licht mit der Menge der in der Probe vorhandenen Nukleinsäure korreliert werden kann, und wobei die Erzeugung von Licht bei 25°C bis 37°C ein Maximum in weniger als 15 Minuten erreicht.

11. Kit zum Erfassen des Vorhandenseins und/oder der Menge eines Analyts in einer Probe, umfassend:
(a) die Verbindung nach Anspruch 1, und
(b) einen Puffer.

## Revendications

1. Composé ayant l'une des structures suivantes 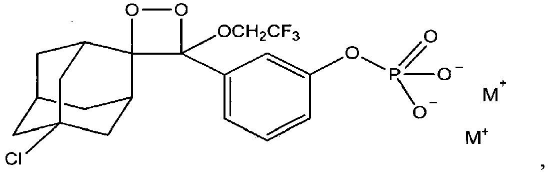 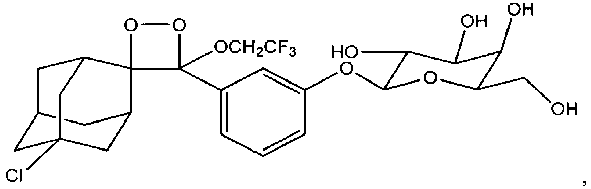 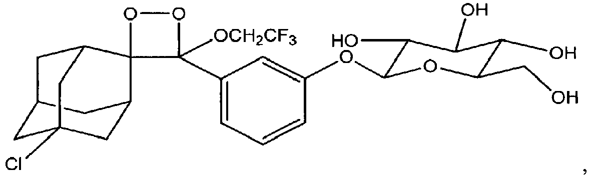 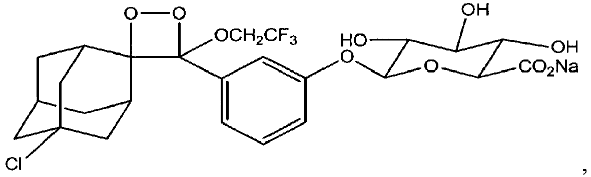 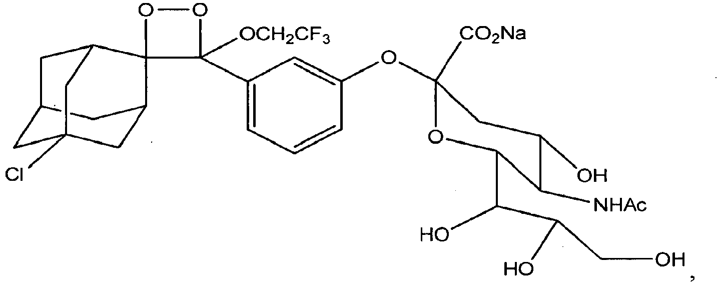 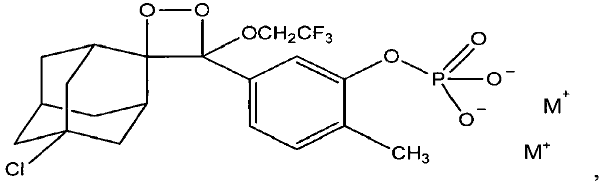 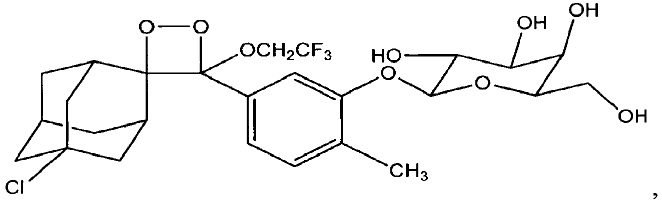 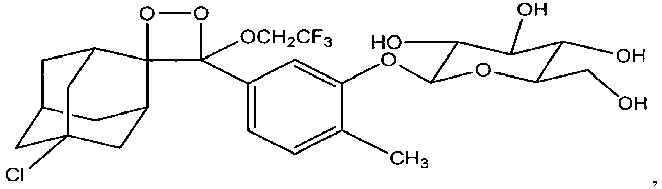 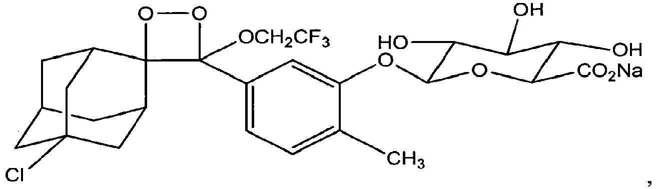 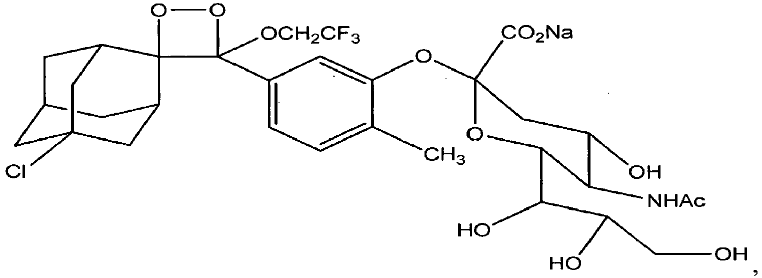 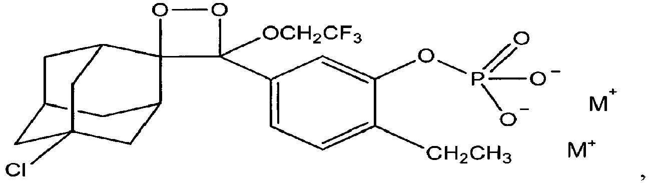 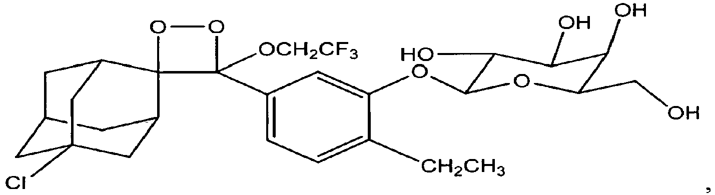 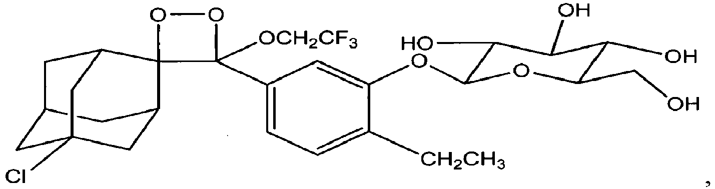 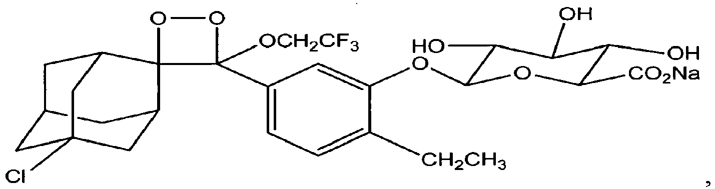 or 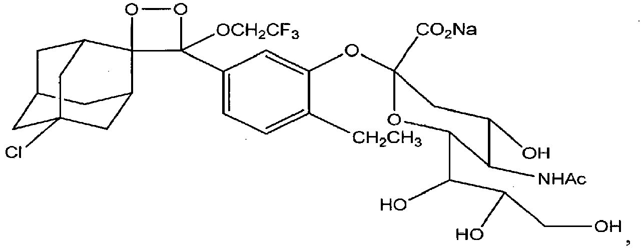 M⁺ représentant un cation d'un métal alcalin, par exemple le sodium ou le potassium, l'ammonium, ou un alkyle en C₁₋₇, un aralkyle ou un cation ammonium quaternaire aromatique, N(D₃)₄⁺ dans lequel chaque D₃ peut être un alkyle, par exemple un méthyle ou un éthyle, un aralkyle, par exemple un benzyle ou faire partie d'un système cyclique hétérocyclique, par exemple le pyridinium ou en particulier le sel de disodium.

2. Procédé de génération de lumière, comprenant les étapes suivantes :
(a) utilisation du composé selon la revendication 1 ;
(b) utilisation d'un complexe enzymatique comprenant une fraction enzymatique qui est
(c) capable de cliver le composé ;mise en contact du complexe enzymatique avec le composé pour former un mélange réactionnel ; et
(d) laisser le mélange réactionnel générer de la lumière.

3. Procédé selon la revendication 2, la fraction enzymatique comprenant une enzyme hydrolytique.

4. Procédé selon la revendication 3, l'enzyme hydrolytique étant une phosphatase alcaline, la β-galactosidase, la β-glucosidase, la β-glucuronidase ou la neuraminidase.

5. Procédé selon la revendication 4, comprenant en outre l'étape de détection de la lumière générée par le mélange réactionnel après l'ajout du composé, la génération de lumière indiquant la présence de l'enzyme et la quantité de lumière générée peut être corrélée à la quantité de l'enzyme présente dans l'échantillon, et la génération de lumière comprise entre 25°C et 37°C atteignant un maximum en moins de 15 minutes.

6. Procédé selon la revendication 2, le mélange réactionnel comprenant en outre un amplificateur comprenant un sel d'ammonium quaternaire polymère, un sel de phosphonium quaternaire polymère ou une combinaison de ces derniers.

7. Procédé selon la revendication 6, l'amplificateur comprenant en outre un colorant accepteur.

8. Procédé de dosage pour déterminer la présence et/ou la quantité d'une enzyme dans un échantillon, comprenant les étapes suivantes :
(a) utilisation du composé selon la revendication 1 ;
(b) utilisation d'un échantillon soupçonné de contenir l'enzyme qui est capable de cliver le composé de sorte qu'il se décompose et génère de la lumière
(c) mise en contact de l'échantillon avec le composé pour former un mélange réactionnel
(d) détection de la lumière générée par le mélange réactionnel après l'ajout du composé, la génération de lumière indiquant la présence de l'enzyme et la quantité de lumière générée peut être corrélée à la quantité de l'enzyme présente dans l'échantillon, et la génération de lumière comprise entre 25°C et 37°C atteignant un maximum en moins de 15 minutes.

9. Procédé de dosage pour déterminer la présence et/ou la quantité d'un antigène dans un échantillon, comprenant les étapes suivantes :
(a) utilisation du composé selon la revendication 1 ;
(b) utilisation d'un échantillon soupçonné de comprendre l'antigène ;
(c) utilisation d'un antigène lié à une enzyme comprenant l'antigène et une enzyme capable de cliver le composé de sorte qu'il se décompose et génère de la lumière ;
(d) utilisation d'une phase solide comprenant un anticorps capable de se lier à l'antigène ;
(e) mise en contact de l'échantillon et d'un antigène lié à une enzyme avec la phase solide pour former un complexe enzymatique ;
(f) mise en contact du complexe enzymatique avec le composé pour former un mélange
(g) réactionnel mise en contact du complexe enzymatique avec le composé pour former un mélange réactionnel; et, détection de la lumière générée par le mélange réactionnel après l'ajout du
composé, la génération de lumière indiquant la présence de l'antigène et la quantité de lumière générée peut être corrélée à la quantité de l'antigène présente dans l'échantillon et la génération de lumière comprise entre 25°C et 37°C atteignant une quantité maximale en moins de 15 minutes.

10. Procédé de dosage pour déterminer la présence et/ou la quantité d'un acide nucléique dans un échantillon, comprenant les étapes suivantes :
(a) utilisation du composé selon la revendication 1 ;
(b) utilisation d'un échantillon soupçonné de comprendre l'acide nucléique ;
(c) immobilisation de l'acide nucléique vis-à-vis d'une phase solide ;
(d) utilisation d'un oligonucléotide lié à une enzyme comprenant un oligonucléotide capable de s'hybrider à l'acide nucléique et une enzyme capable de cliver le composé de sorte qu'il se décompose et génère de la lumière ;
(e) mise en contact de l'acide nucléique immobilisé et d'un oligonucléotide lié à une enzyme pour former un complexe enzymatique ;
(f) mise en contact du complexe enzymatique avec le composé pour former un
(g) mélange réactionnel ; et détection de la lumière générée par le mélange réactionnel après l'ajout du
composé, la génération de lumière indiquant la présence de l'acide nucléique et la quantité de lumière générée peut être corrélée à la quantité de l'acide nucléique présente dans l'échantillon, et la génération de lumière comprise entre 25°C et 37°C atteignant un maximum en moins de 15 minutes.

11. Kit pour détecter la présence et/ou la quantité d'un analyte dans un échantillon comprenant :
(a) le composé selon la revendication 1, et
(b) un tampon.
